# EUROPEAN PATENT SPECIFICATION

(11) **EP 3 000 811 B1**
(45) Date of publication and mention of the grant of the patent: **08.11.2017**
(21) Application number: 14186189.8
(22) Date of filing: 24.09.2014
(51) Int. Cl.: C07D 323/00, C07D 493/08, A61K 31/357

(54) **Crown Ether Complexes and Methods for Poducing the Same**
Kronenetherkomplexe und Methoden ihrer Herstellung
Complexes de Éther Couronne et des Méthodes pour les Produire

(43) Date of publication of application: 30.03.2016
(73) Proprietor: Friedrich-Alexander-Universität Erlangen-Nürnberg, 91054 Erlangen (DE)
(72) Inventor: von Delius, Max, 91058 Erlangen (DE); Brachvogel, René-Chris, 91186 Büchenbach (DE)
(74) Representative: Vossius & Partner Patentanwälte Rechtsanwälte mbB

(56) References cited:
- EP-A1- 2 332 929
- WO-A1-92/05804
- WO-A2-2008/037484

## Description

### Field of the invention

The present invention relates to novel crown ether complexes and methods for producing the same as well as pharmaceutical or diagnostic compositions comprising the same.

### Background of the invention

The discovery of coronands and cryptands, organic compounds that can accommodate metal ions in a preorganized two- or three-dimensional environment, has been a milestone in supramolecular chemistry, leading to countless applications from organic synthesis to metallurgy and medicine. Traditionally, these compounds are prepared via multistep organic synthesis and one of their characteristic features is their high kinetic stability. Here we report the metal-templated self-assembly of a new class of coronates and cryptates, containing two tripodal *O*,*O*,*O*-orthoester motifs. In contrast to their classical analogues, the compounds described herein are constitutionally dynamic in the presence of acid, a property that opens up a wide range of opportunities, from systems chemistry to traceless medicinal metal ion delivery or molecular sensing.

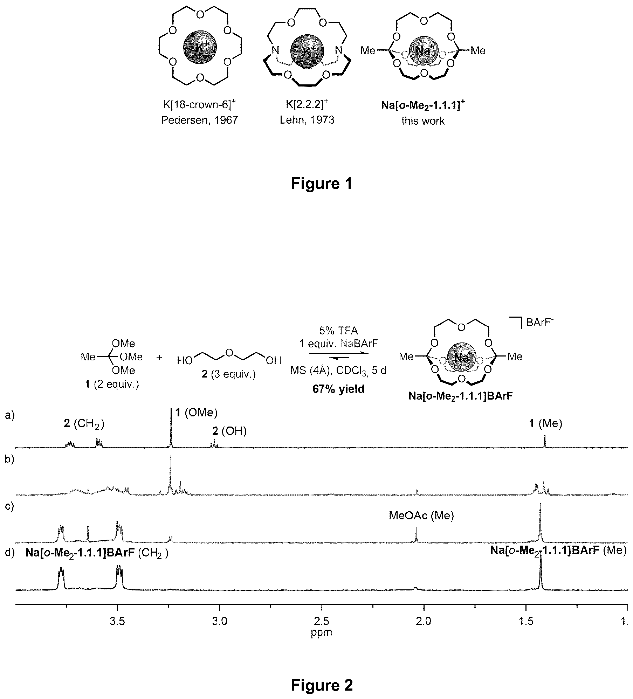

Past progress in supramolecular chemistry has been driven chiefly by the development of new macrocyclic compounds.¹ Pedersen's crown ethers (coronands)² and Lehn's cryptands^{3,4} (Figure 1) are excellent examples of compounds that initially served as platforms for studying non-covalent interactions, but have ultimately found widespread application in industry and medicine.⁵

In the last decade, rationally designed three-dimensional cage compounds⁶⁻⁸ have become larger and larger,⁸⁻¹³ enabling in one extreme case even the accommodation of a small protein.¹⁴ To achieve the self-assembly of such large structures, the method of choice is dynamic constitutional/covalent chemistry (DCC),¹⁵⁻¹⁷ which offers the essential feature of error-correction that is needed to avoid significant by-product formation. Besides providing high yielding syntheses, DCC generally gives rise to target structures that are dynamic and responsive to external stimuli under the conditions of their preparation. In the context of the emerging field of systems chemistry,^{18,19} dynamic macrocycles and cages have served as a valuable testing ground for the investigation and manipulation of complex molecular networks.²⁰⁻²⁵ Constitutionally dynamic cryptates would represent the smallest and simplest platform for studying molecular complexity, but to the best of our knowledge there are no reports of monometallic cryptates²⁶⁻²⁸ that are accessible via reversible reactions.

Botti et al. describe orthoester derivatives of crown ethers as carriers for pharmaceutical and diagnostic compositions⁴¹.

It is an object of the present invention to provide novel crown ether complexes as well as methods for preparing the same. The methods should preferably provide a simple synthetic route to the claimed compounds.

### Cited publications

1. Steed, J. W. & Gale, P. A. (ed.) Supramolecular Chemistry (Wiley-VCH, Weinheim, Germany, 2012).
2. Pedersen, C. J. Cyclic polyethers and their complexes with metal salts. J. Am. Chem. Soc. 89, 7017-7036 (1967).
3. Dietrich, B., Lehn, J.-M. & Sauvage, J.-P. Cryptates - XI. Complexes macrobicycliques, formation, structure, propriétés. Tetrahedron 29, 1647-1658 (1973).
4. Lehn, J.-M. Cryptates: the chemistry of macropolycyclic inclusion complexes. Acc. Chem. Res. 11, 49-57 (1978).
5. Schneider, H.-J. (ed.) Applications of Supramolecular Chemistry (CRC Press, Boca Raton, USA, 2012).
6. Harris, K., Fujita, D. & Fujita, M. Giant hollow MnL2n spherical complexes: structure, functionalisation and applications. Chem. Commun. 49, 6703-6712 (2013).
7. Zhang, G. & Mastalerz, M. Organic cage compounds - from shape-persistency to function. Chem. Soc. Rev. 43, 1934-1947 (2014).
8. Han, M., Engelhard, D. M. & Clever, G. H. Self-assembled coordination cages based on banana-shaped ligands. Chem. Soc. Rev. 43, 1848-1860 (2014).
9. Sun, Q. F. et al. Self-assembled M24L48 polyhedra and their sharp structural switch upon subtle ligand variation. Science 328, 1144-1147 (2010).
10. Tozawa, T. et al. Porous organic cages. Nature Mat. 8, 973-978 (2009).
11. Granzhan, A. et al. Connection of metallamacrocycles via dynamic covalent chemistry: a versatile method for the synthesis of molecular cages J. Am. Chem. Soc. 133, 7106-7115 (2011).
12. Bilbeisi, R. A. et al. Subcomponent self-assembly and guest-binding properties of face-capped Fe4L48+ capsules. J. Am. Chem. Soc. 134, 5110-5119 (2012).
13. Zhang, G., Presly, O., White, F., Oppel, I. M. & Mastalerz, M. A permanent mesoporous organic cage with an exceptionally high surface area. Angew. Chem. Int. Ed. 53, 1516-1520 (2014).
14. Fujita, D. et al. Protein encapsulation within synthetic molecular hosts. Nature Commun. 3, 1093-1099 (2012).
15. Corbett, P. T. et al. Dynamic combinatorial chemistry. Chem. Rev. 106, 3652-3711 (2006).
16. Jin, Y., Yu, C., Denman, R. J. & Zhang, W. Recent advances in dynamic covalent chemistry. Chem. Soc. Rev. 42, 6634-6654 (2013).
17. Herrmann, A. Dynamic combinatorial/covalent chemistry: a tool to read, generate and modulate the bioactivity of compounds and compound mixtures. Chem. Soc. Rev. 43, 1899-1933 (2014).
18. Ludlow, F. R. & Otto, S. Systems chemistry. Chem. Soc. Rev. 37, 101-108 (2008).
19. Li, J. Nowak, P. & Otto, S. Dynamic combinatorial libraries: from exploring molecular recognition to systems chemistry. J. Am. Chem. Soc. 135, 9222-9239 (2013).
20. Carnall, J. M. A. et al. Mechanosensitive self-replication driven by self-organization. Science 327, 1502-1506 (2010)
21. Zarra, S., Wood, D. M., Roberts, D. A. & Nitschke, J. R. Molecular containers in complex chemical systems. Chem. Soc. Rev. (2014) DOI: 10.1039/C4CS00165F.
22. I. A. Riddell, I. A. et al. Anion-induced reconstitution of a self-assembling system to express a chloride-binding Co10L15 pentagonal prism. Nature Chem. 4, 751-756 (2012).
23. Han, M. et al. Light-triggered guest uptake and release by a photochromic coordination cage. Angew. Chem. Int. Ed. 52, 1319-1323 (2013).
24. S. Hiraoka, K. Harano, M. Shiro and M. Shionoya, Quantitative Dynamic Interconversion between Agl-Mediated Capsule and Cage Complexes Accompanying Guest Encapsulation/Release. Angew. Chem. Int. Ed. 44, 2727-2731 (2005).
25. A. R. Stefankiewicz, M. R. Sambrook and J. K. M. Sanders, Template-directed synthesis of multi-component organic cages in water. Chem. Sci. 3, 2326-2329 (2012).
26. Jazwinski, J. et al. Polyaza macrobicyclic cryptands: synthesis, crystal structures of a cyclophane type macrobicyclic cryptand and of its dinuclear copper(I) cryptate, and anion binding features. J. Chem. Soc., Chem. Commun. 1691-1694 (1987).
27. MacDowell, D. & Nelson, J. Facile synthesis of a new family of cage molecules. Tetrahedron Lett. 29, 385-386 (1988).
28. Howarth, O. W., Morgan, G. G., McKee, V. & Nelson, J. Conformational choice in disilver cryptates; an 1H NMR and structural study. J. Chem. Soc., Dalton Trans. 2097-2102 (1999).
29. Lightburn, T. E., Dombrowski, M. T. & Tan, K. L. Catalytic scaffolding ligands: an efficient strategy for directing reactions. J. Am. Chem. Soc.130, 9210-9211 (2008).
30. Tan, K. L. Induced intramolecularity: an effective strategy in catalysis. ACS Catal. 1, 877-886 (2011).
31. DeWolfe, R. H. Synthesis of carboxylic and carbonic ortho esters. Synthesis 153-172 (1974).
32. Lin, Y.-H., Lai, C.-C., Liu, Y.-H., Peng, S.-M. & Chiu, S.-H. Sodium ions template the formation of rotaxanes from BPX26C6 and nonconjugated amide and urea functionalities. Angew. Chem. Int. Ed. 52, 10231-10236 (2013).
33. Wu, K.-D., Lin, Y.-H., Lai, C.-C. & Chiu, S.-H. Na+ ion templated threading of oligo(ethylene glycol) chains through BPX26C6 allows synthesis of [2]rotaxanes under solvent-free conditions. Org. Lett. 16, 1068-1071 (2014).
34. Rosenthal, M. R. The myth of the non-coordinating anion. J. Chem. Educ. 50, 331-335 (1973).
35. Lee, S., Chen, C.-H. & Flood, A. H. A pentagonal cyanostar macrocycle with cyanostilbene CH donors binds anions and forms dialkylphosphate [3]rotaxanes. Nature Chem. 5, 704-710 (2013).
36. Gold, V. & Sghibartz, C. M. Crown ether acetals: detection of cation binding by kinetic measurements. J. Chem. Soc., Chem. Commun. 507-508 (1978).
37. Gold, V. & Sghibartz, C. M. Crown ether acetals: synthesis and characterisation of a family of novel oxygen macrocyclic compounds. J. Chem. Soc., Perkin Trans. I 453-457 (1983).
38. Baker, D. S., Gold, V. & Sghibartz, C. M. The influence of cation binding on the kinetics of the hydrolysis of crown ether acetals. J. Chem. Soc., Perkin Trans. II 1121-1128 (1983).
39. Moras, P.D. & Weiss, R. Etude structurale des cryptates. III. Structure cristalline et moléculaire du cryptate de sodium C18H36N2O6.Nal. Acta Cryst. B29, 396-399 (1973).
40. Shannon. R. D. Revised effective ionic radii and systematic studies of interatomic distances in halides and chalcogenides. Acta Cryst. A32, 751-767 (1976).
41. Botti, P., Tchertchian, S. & Theurillat, D. Orthoester derivatives of crown ethers as carriers for pharmaceutical and diagnostic compositions. Eur. Pat. Appl. EP 2 332 929 A1 (2009).
42. Crossing, I., Raabe, I. Noncoordinating anions - fact or fiction? A survey of likely candidates. Angew. Chem. Int. Ed. 43, 2066-2090 (2004).
43. Breck, D. W., Eversole, W. G., Milton, R. M., Reed, T. B., Thomas, T. L. Crystalline zeolites. I. The properties of a new synthetic zeolite, type A. J. Am. Chem. Soc. 78, 5963-5972, (1956).

### Brief description of the figures

Figure 1 shows a comparison of specific compounds prepared by Pedersen, Lehn and the present inventors.
Figure 2 shows ¹H NMR spectra obtained in the treatment of a chloroform solution of trimethyl orthoacetate (1) and diethylene glycol (2) with catalyst trifluoroacetic acid (TFA) and stoichiometric metal template.
Figure 3 shows the solid state structure of **Na[*o*-Me₂-1.1.1]BArF.**
Figure 4 shows the structures of four representative compounds which can be obtained as products using the described method.

### Definitions

The following definitions apply throughout unless specified otherwise.

The term "alkyl" refers to a saturated straight or branched hydrocarbon chain. Throughout the present application the alkyl group independently for each occurrence preferably has 1 to 12, more preferably 1 to 6, even more preferably 1 to 4 carbon atoms.

The term "alkenyl" refers to a straight or branched hydrocarbon chain which includes at least one C=C double bond. Throughout the present application the alkenyl group independently for each occurrence preferably has 2 to 12, more preferably 2 to 6, even more preferably 2 to 4 carbon atoms.

The term "alkynyl" refers to a straight or branched hydrocarbon chain which includes at least one C=C triple bond. Throughout the present application the alkynyl group independently for each occurrence preferably has 2 to 12, more preferably 2 to 6, even more preferably 2 to 4 carbon atoms.

The term "aryl" preferably refers to an aromatic ring or ring system. The term "aryl" is also meant to include bicyclic, tricyclic and polycyclic versions thereof. Throughout the present application the aryl group independently for each occurrence usually has 5 to 20, more usually 5 to 14, preferably 5 to 12, more preferably 5 to 7, even more preferably 6 carbon atoms in the ring or ring system. Examples are phenyl, naphthyl, anthracenyl, tetracenyl, pentacenyl, pyrenyl and perylenyl, preferably naphthyl, anthracenyl and phenyl, even more preferably phenyl.

The term "cycloalkyl" represents a cyclic version of "alkyl". The term "cycloalkyl" is also meant to include bicyclic, tricyclic and polycyclic versions thereof. Throughout the present application the cycloalkyl group independently for each occurrence usually has 5 to 20, more usually 5 to 14, preferably 5 to 12, more preferably 5 to 7, even more preferably 6 carbon atoms in the ring or ring system.

The term "carbocyclyl" or "carbocyclic" covers any hydrocarbon ring or ring system which does not include heteroatoms in the ring or ring system. The term "carbocyclyl ring" covers saturated (including cycloalkyl rings), unsaturated rings and aromatic rings (including aryl rings). The term "carbocyclyl" is also meant to include bicyclic, tricyclic and polycyclic versions thereof. Throughout the present application the carbocyclyl group independently for each occurrence usually has 5 to 20, more usually 5 to 14, preferably 5 to 12, more preferably 5 to 7, even more preferably 6 carbon atoms in the ring or ring system.

"Hal" or "halogen" represents F, Cl, Br and I, preferably F or Cl.

The term "heteroaryl" preferably refers to an aromatic ring or ring system wherein one or more of the carbon atoms in the ring have been replaced by 1, 2, 3, or 4 (for the five-membered ring), 1, 2, 3, 4, or 5 (for the six-membered ring), etc. of the same or different heteroatoms, whereby the heteroatoms are selected from O, N and S. Examples of the heteroaryl group include triazole, benzotriazole, thiophenyl, pyrrole, furan, imidazole, pyrazole, oxazole, thiazole, and pyridine. Preferred examples include triazole, benzotriazole, and thiophenyl. Throughout the present application the heteroaryl group independently for each occurrence usually has 5 to 20, more usually 5 to 14, preferably 5 to 12, more preferably 5 to 7 atoms in the ring or ring system.

The term "heterocyclyl" or "heterocyclic" covers any ring or ring system wherein at least one of the carbon atoms in the ring has been replaced by 1, 2, 3, or 4 (for the five-membered ring), 1, 2, 3, 4, or 5 (for the six-membered ring), etc. of the same or different heteroatoms, whereby the heteroatoms are selected from O, N and S. The term "heterocyclyl ring" covers saturated, unsaturated rings and aromatic rings (including heteroaryl rings). The term "heterocyclyl" is also meant to include bicyclic, tricyclic and polycyclic versions thereof, wherein at least one of the rings includes at least one heteroatom. Examples include triazole, benzotriazole, thiophenyl, pyrrole, pyrrolidine, oxolane, furan, imidazolidine, imidazole, pyrazole, oxazolidine, oxazole, thiazole, piperidine, pyridine, morpholine, piperazine, and dioxolane as well as ring systems including at least one of these rings. Preferred examples include triazole, benzotriazole and thiophenyl. Throughout the present application the heterocyclyl group independently for each occurrence usually has 5 to 20, more usually 5 to 14, preferably 5 to 12, more preferably 5 to 7 atoms in the ring or ring system.

The term "non-coordinating anion" (NCA) refers to tetraarylborate anions B(Ar¹)₄, aluminate anions Al(OR¹⁰)₄ or (R¹⁰O)₃Al-F-Al(OR¹⁰)₃ and carborane anions R¹¹-CB₁₁R¹²₁₁.

Unless mentioned otherwise, if a compound or moiety is referred to as being "optionally substituted", it can in each instance include one or more of the indicated substituents, whereby the substituents can be the same or different.

The alkyl, alkenyl and alkynyl group can be optionally substituted with one or more substituents which are independently for each occurrence selected from -halogen, -CN, -NR²⁰R²⁰, -N₃, -OC(O)-R²⁰ -C(O)-O-R²⁰, -N(H)C(O)-R²⁰, -C(O)-N(H)-R²⁰, -C(O)-R²⁰, -S-R²⁰, -O-R²⁰, -(optionally substituted carbocyclyl) (wherein the optional substituent is, for example, selected from -Hal, -OH, -N₃, -CN, -COOH, -alkene, and -alkyne), -(optionally substituted heterocyclyl) (wherein the optional substituent is, for example, selected from -Hal, -OH, -N₃, -CN, -COOH, -alkene, and -alkyne), -alkene (e.g., -CH=CH₂), and -alkyne (e.g., -C=CH). Preferably, the optional substituent of the alkyl, alkenyl and alkynyl group is independently selected from -halogen, -CN, -N₃, -OC(O)-R²⁰, -C(O)-O-R²⁰, -N(H)C(O)-R²⁰, -C(O)-N(H)-R²⁰, -O-R²⁰, -(optionally substituted carbocyclyl), -(optionally substituted heterocyclyl), -alkene, and -alkyne.

The heterocyclyl group and carbocyclyl group can be optionally substituted with one or more substituents which are independently for each occurrence selected from -halogen, -CN, -NR²⁰R²⁰, -(CH₂)ₛ-N₃, -OC(O)-R²⁰, -C(O)-O-R²⁰, -N(H)C(O)-R²⁰, -C(O)-N(H)-R²⁰, -OR²⁰, -SR²⁰, -(optionally substituted alkyl) (wherein the optional substituent is, for example, selected from -Hal, -OH, -N₃, -CN, -COOH, -alkene, and -alkyne), -alkene, -alkyne, -(optionally substituted carbocyclyl) (wherein the optional substituent is, for example, selected from -Hal, -OH, -N₃, -CN, -COOH, -alkene, and -alkyne), -alkyl-(optionally substituted carbocyclyl) (wherein the optional substituent is, for example, selected from -Hal, -OH, -N₃, -CN, -COOH, -alkene, and -alkyne), -(optionally substituted heterocyclyl) (wherein the optional substituent is, for example, selected from -Hal, -OH, -N₃, -CN, -COOH, -alkene, and -alkyne), and -alkyl-(optionally substituted heterocyclyl) (wherein the optional substituent is, for example, selected from -Hal, -OH, -N₃, -CN, -alkene, and -alkyne). Preferably, the optional substituent of the heterocyclyl group and carbocyclyl group is independently selected from -halogen, -CN, -(CH₂)ₛ-N₃, -OC(O)-R²⁰, -C(O)-O-R²⁰, -N(H)C(O)-R²⁰, -C(O)-N(H)-R²⁰, -OR²⁰, -SR²⁰, -(alkyl which is substituted by halogen), -alkene, -alkyne, -(optionally substituted carbocyclyl) and -(optionally substituted heterocyclyl).

**R**²⁰ is independently for each occurrence selected from -H, -(optionally substituted alkyl), -(optionally substituted carbocyclyl), -alkyl-(optionally substituted carbocyclyl), -(optionally substituted heterocyclyl), and -alkyl-(optionally substituted heterocyclyl), preferably R²⁰ is independently for each occurrence selected from -H, -(optionally substituted alkyl), -(optionally substituted carbocyclyl), and -(optionally substituted heterocyclyl).

s is preferably 0 to 12, more preferably 0 to 6.

### Detailed description of the invention

The present invention relates to a method comprising the step of reacting at least one compound having the formula (I) 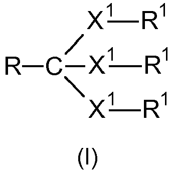 with at least one compound having the formula (II) 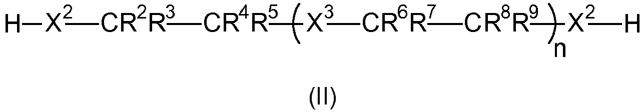 in the presence of at least one salt having the formula (IIIa)

M¹Y¹ (IIIa)

The exact reaction mechanism between the compound having the formula (I), the compound having the formula (II) and the salt having the formula (IIIa) is not known. However, a monocyclic compound having the formula (VI) can be detected in the reaction mixture, particularly if the salt having the formula (IIIa) is not present.

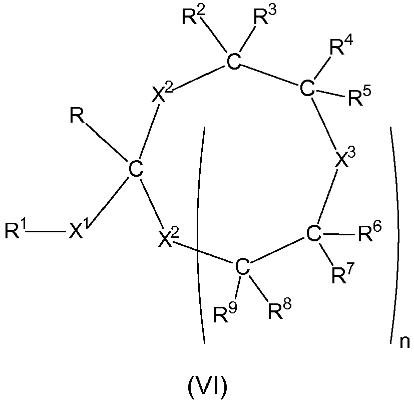

The NCA complexes having the formula (V-i) and (V-ii) 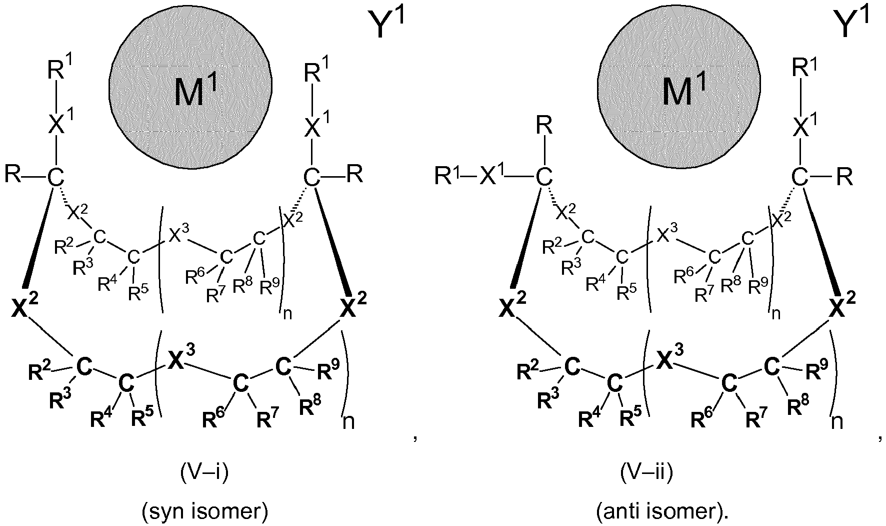 can be detected in the reaction mixture as the reaction proceeds.

The compounds are conformational isomers and will sometimes be collectively referred to as NCA complex having the formula (V) in the following. These NCA complexes having the formula (V) are stable in the absence of acid and can be isolated from the reaction mixture and identified.

After a longer duration of the reaction, the concentration of the NCA complex having the formula (V) decreases and a NCA complex having the formula (IV) can be detected

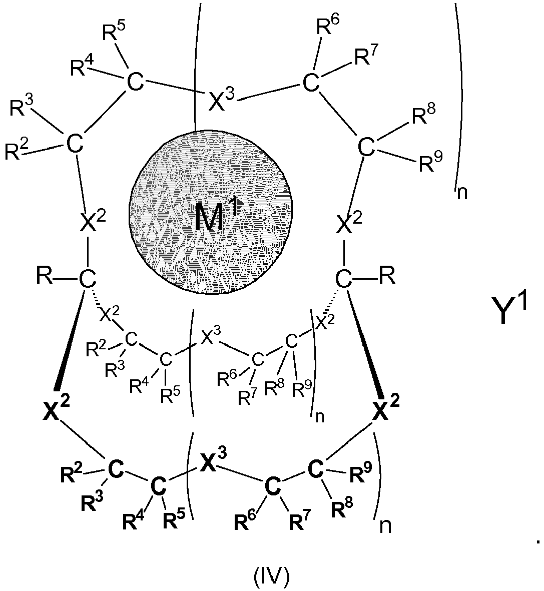

This sequence of reaction steps can be watched by analyzing samples of the reaction mixture at regular intervals during the duration of the reaction.

### Compound having the formula (I)

In the method of the present invention a compound having the formula (I) is employed 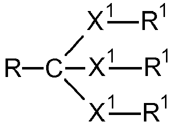 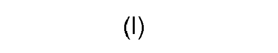
- **X**¹: is independently for each occurrence selected from the group consisting of O and S; preferably X¹ is O.
- **R**¹: is independently for each occurrence selected from the group consisting of alkyl and carbocyclyl. Preferably, R¹ is independently for each occurrence selected from the group consisting of alkyl and aryl, more preferably, R¹ is independently for each occurrence selected from the group consisting of alkyl, even more preferably R¹ is CH₃.
The group R is not particularly restricted and can be any moiety which does not detrimentally interfere with the claimed method. The group R is also particularly suitable for labeling the compounds of the present invention. Therefore, it is possible to employ any substituent which is suitable as a label. Examples thereof include radioactive labels, fluorescent labels, spin labels and near-infrared labels.
In one embodiment, **R** is selected from the group consisting of H, alkyl, alkenyl, alkinyl, carbocyclyl and heterocyclyl, wherein the alkyl, alkenyl, alkinyl, carbocyclyl and heterocyclyl can be optionally substituted.
R is preferably selected from the group consisting of H, alkyl, alkinyl, carbocyclyl and heterocyclyl, more preferably R is selected from the group consisting of H, alkyl and carbocyclyl, even more preferably R is selected from the group consisting of H, alkyl (e.g. C₁₋₄ alkyl), and aryl (e.g., phenyl and anthracenyl).
The alkyl, alkenyl and alkynyl group can be optionally substituted with one or more substituents which are independently for each occurrence selected from -halogen, -CN, -NR²⁰R²⁰, -N₃, -OC(O)-R²⁰, -C(O)-O-R²⁰, -N(H)C(O)-R²⁰, -C(O)-N(H)-R²⁰, -C(O)-R²⁰, -S-R²⁰, -O-R²⁰, -(optionally substituted carbocyclyl) (wherein the optional substituent is, for example, selected from -Hal, -OH, -N₃, -CN, -COOH, -alkene, and -alkyne), -(optionally substituted heterocyclyl) (wherein the optional substituent is, for example, selected from -Hal, -OH, -N₃, -CN, -COOH, -alkene, and -alkyne), -alkene (e.g., -CH=CH₂), and -alkyne (e.g., -C≡CH). Preferably, the optional substituent of the alkyl, alkenyl and alkynyl group is independently selected from -halogen, -CN,-N₃, -OC(O)-R²⁰, -C(O)-O-R²⁰, -N(H)C(O)-R²⁰, -C(O)-N(H)-R²⁰, -O-R²⁰, -(optionally substituted carbocyclyl), -(optionally substituted heterocyclyl), -alkene, and -alkyne.
The heterocyclyl group and carbocyclyl group can be optionally substituted with one or more substituents which are independently for each occurrence selected from -halogen, -CN, -NR²⁰R²⁰, -(CH₂)ₛ-N₃, -OC(O)-R²⁰, -C(O)-O-R²⁰, -N(H)C(O)-R²⁰, -C(O)-N(H)-R²⁰, -OR²⁰, -SR²⁰, -(optionally substituted alkyl) (wherein the optional substituent is, for example, selected from -Hal, -OH, -N₃, -CN, -COOH, -alkene, and -alkyne), -alkene, -alkyne, -(optionally substituted carbocyclyl) (wherein the optional substituent is, for example, selected from -Hal, -OH, -N₃, -CN, -COOH, -alkene, and -alkyne), -alkyl-(optionally substituted carbocyclyl) (wherein the optional substituent is, for example, selected from -Hal, -OH, -N₃, -CN, -COOH, -alkene, and -alkyne), -(optionally substituted heterocyclyl) (wherein the optional substituent is, for example, selected from -Hal, -OH, -N₃, -CN, -COOH, -alkene, and -alkyne), and -alkyl-(optionally substituted heterocyclyl) (wherein the optional substituent is, for example, selected from -Hal, -OH, -N₃, -CN, -COOH, -alkene, and -alkyne). Preferably, the optional substituent of the heterocyclyl group and carbocyclyl group is independently selected from -halogen, -CN, -(CH₂)ₛ-N₃, -OC(O)-R²⁰, -C(O)-O-R²⁰, -N(H)C(O)-R²⁰, -C(O)-N(H)-R²⁰, -OR²⁰, -SR²⁰, -(alkyl which is substituted by halogen), -alkene, -alkyne, -(optionally substituted carbocyclyl) and -(optionally substituted heterocyclyl).
- **R²⁰**: is independently for each occurrence selected from -H, -(optionally substituted alkyl), -(optionally substituted carbocyclyl), -alkyl-(optionally substituted carbocyclyl), -(optionally substituted heterocyclyl), and -alkyl-(optionally substituted heterocyclyl), preferably R²⁰ is independently for each occurrence selected from -H, -(optionally substituted alkyl), -(optionally substituted carbocyclyl), and -(optionally substituted heterocyclyl).
In one embodiment, R is preferably independently for each occurrence selected from the group consisting of -H, -alkyl, -alkyl-OH, -alkyl-N₃, -alkyl-C≡CH, -alkyl-C(H)=CH₂, -alkyl-1,2,3-triazolyl-4-alkyl, -alkyl-1,2,3-triazolyl-4-aryl, -alkyl-1,2,3-triazolyl-1-alkyl, -alkyl-1,2,3-triazolyl-1-aryl, -alkyl-CN, -alkyl-COOH, -alkyl-O-alkyl, -alkyl-O-aryl, -alkyl-OC(O)-alkyl, -alkyl -C(O)-O-alkyl, -alkyl-N(H)C(O)-alkyl, -alkyl-C(O)-N(H)-alkyl, -alkyl-COOH, -aryl-OHaryl-N₃, -aryl-CN, -aryl-COOH, -aryl-F, -aryl-Cl, -aryl-Br, -aryl-(alkyl which is substituted by halogen), -aryl-I, -aryl-C≡CH, -aryl-C(H)=CH₂, -aryl-1,2,3-triazolyl-4-C₁₋₁₂ alkyl, -aryl-1,2,3-triazolyl-4-aryl, -aryl-1,2,3-triazolyl-1-alkyl, -aryl-1,2,3-triazolyl-1-aryl, -aryl-O-alkyl, -aryl-O-aryl, -aryl-OC(O)-alkyl, -aryl-C(O)-O-alkyl, -aryl-N(H)C(O)-alkyl, -aryl-C(O)-N(H)-alkyl, -aryl-COOH, and 9-anthracenyl.

Examples of compounds having the formula (I) include, but are not limited to, (H₃C)C(OCH₃)₃, HC(OCH₃)₃, (H₃C-H₂C)C(OCH₃)₃, (C₆H₅)C(OCH₃)₃, HC(OC₆H₅)₃, (H₃C)C(SCH₃)₃, HC(SCH₃)₃, (H₃C-H₂C)C(SCH₃)₃, (C₆H₅)C(SCH₃)₃, and HC(SC₆H₅)₃.

In the method of the present invention a single compound having the formula (I) or a mixture of compounds having the formula (I) can be employed.

### Compound having the formula (II)

In the method of the present invention a compound having the formula (II) is also employed 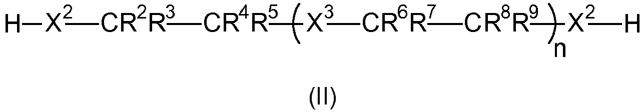
- **X**²: is independently for each occurrence selected from the group consisting of O and S. Preferably X² is O.
- **X**³: is independently for each occurrence selected from the group consisting of O and S. Preferably, X³ is O.
- **R**², **R**³, **R**⁴, **R**⁵, **R**⁶, **R**⁷, **R**⁸, and **R**⁹: are independently for each occurrence selected from the group consisting of H, optionally substituted carbocyclyl and optionally substituted alkyl or any two of R², R³, R⁴, R⁵, R⁶, R⁷, R⁸, and R⁹ which are attached to the same carbon atom or two adjacent carbon atoms can be bonded to form a 3- to 7-membered carbocyclic ring. In one preferred embodiment, R², R³, R⁴, R⁵, R⁶, R⁷, R⁸, and R⁹ are H. In another embodiment, R², R³, R⁴, R⁵, R⁶, R⁷, R⁸, and R⁹ are independently for each occurrence selected from the group consisting of H and alkyl, typically R⁵ and R⁶ are alkyl in this embodiment and the others are H. In a further embodiment, at least two of R², R³, R⁴, R⁵, R⁶, R⁷, R⁸, and R⁹ (typically 2 or 4 of R², R³, R⁴, R⁵, R⁶, R⁷, R⁸, and R⁹) which are attached to the same carbon atom or two adjacent carbon atoms can be bonded to form a 3- to 12-membered carbocyclic ring and the others are H. With respect to R², R³, R⁴, R⁵, R⁶, R⁷, R⁸, and R⁹ the 3- to 12-membered carbocyclic ring can, for example, be a 3- to 7-membered carbocyclic ring or a 6-membered carbocyclic ring such as a phenyl ring or a cyclohexyl ring.
The alkyl and the carbocyclyl group can be optionally substituted as described above.
The groups **R**², **R**³, **R**⁴, **R**⁵, **R**⁶, **R**⁷, **R**⁸, and **R**⁹ are also suitable for labeling the compounds of the present invention. Therefore, it is possible to employ any substituent which is suitable as a label. Examples thereof include radioactive labels, fluorescent labels, spin labels and near-infrared labels.
- **n**: is independently for each occurrence selected from the group consisting of 0, 1, 2 and 3. Preferably, n is 0, 1, or 2; more preferably, n is 1.

Examples of compounds having the formula (II) include, but are not limited to, HO-CH₂CH₂-(OCH₂CH₂)-OH, HO-CH₂CH₂-(OCH₂CH₂)₂-OH, HO-CH₂CH₂-OH, HO-CH₂CH(R*)-(OCH(R*)CH₂)-OH, HO-CH₂CH(R*)-(OCH(R*)CH₂)₂-OH, (with R* = alkyl), HO-CH₂CH₂-(SCH₂CH₂)-OH, HO-CH₂CH₂-(SCH₂CH₂)₂-OH, HS-CH₂CH₂-(OCH₂CH₂)-SH, HS-CH₂CH₂-(OCH₂CH₂)₂-SH, HS-CH₂CH₂-SH, HS-CH₂CH₂-(SCH₂CH₂)-SH, HS-CH₂CH₂-(SCH₂CH₂)₂-SH, 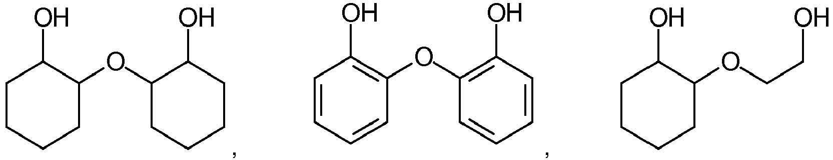 and

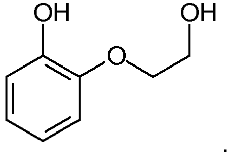

Preferably, compounds having the formula (II) are HO-CH₂CH₂-(OCH₂CH₂)-OH, HO-CH₂CH₂-(OCH₂CH₂)₂-OH and HO-CH₂CH₂-OH. More preferably, compound having the formula (II) is HO-CH₂CH₂-(OCH₂CH₂)-OH.

In the method of the present invention a single compound having the formula (II) or a mixture of compounds having the formula (II) can be employed.

### Salt having the formula (IIIa)

A salt having the formula (IIIa) is employed in the method of the present invention.

M¹Y¹ (IIIa)

- **M**¹: is a cation. The cation is not particularly limited and will typically depend on the desired use of the compounds of the present invention. Examples of suitable cations include, but are not limited to, alkali metals (e.g., Li, Na, K, Rb, Cs), alkali earth metals (e.g., Be, Mg, Ca, Sr, Ba, Ra), group 13 metals (e.g., Al, Ga, In, Tl), group 14 metals (e.g., Ge, Sn, Pb), transition metals of the groups 3 to 12 of the periodic table of elements (e.g., Sc, Y, Ti, Zr, Hf, V, Nb, Ta, Cr, Mo, W, Mn, Tc, Re, Fe, Ru, Os, Co, Rh, Ir, Ni, Pd, Pt, Cu, Ag, Au, Zn, Cd, Hg) and ammonium ions NR^{‡}₄, wherein R^{‡} is independently for each occurrence selected from the group consisting of H and alkyl. Preferably M¹ is selected from alkali metals and alkali earth metals, more preferably M¹ is selected from Li, Na, K, Mg, and Ca, even more preferably M¹ is Na.
- **Y**¹: is a non-coordinating anion (NCA) selected from the group consisting of tetraarylborate anions B(Ar¹)₄, aluminate anions Al(OR¹⁰)₄ or (R¹⁰O)₃Al-F-Al(OR¹⁰)₃ and carborane anions R¹¹-CB₁₁R¹²₁₁. Preferably, the NCA is a tetraarylborate anion B(Ar¹)₄.

In the formula **B(Ar¹)₄** Ar¹ is independently for each occurrence selected from the group consisting of an optionally substituted aryl or optionally substituted heteroaryl group. Examples of Ar¹ include, but are not limited to, 1-imidazolyl, 2-thiophenyl, phenyl, which can each be optionally substituted. Examples of the optional substituent include halogen, cyano, aryl and alkyl which is optionally substituted by halogen (preferably F or Cl) or which is optionally substituted by O-alkyl (preferably OCH₃).

Examples of Ar¹ include, but are not limited to, 1-imidazolyl, 2-thiophenyl, phenyl, phenyl which is substituted by 1 to 5 halogen atoms (preferably F or Cl) and phenyl which is substituted by 1 to 5 trifluoromethyl groups. Specific examples include, but are not limited to, phenyl, 4-halogenophenyl, 3,5-dihalogenophenyl, pentahalogenophenyl, (4-trifluoromethyl)phenyl, 3,5-bis(trifluoromethyl)phenyl, and penta(trifluoromethyl)phenyl, wherein halogeno is preferably F or Cl.

In the formulae **Al(OR¹⁰)₄** or **(R¹⁰O)₃Al-F-Al(OR¹⁰)₃** R¹⁰ is independently for each occurrence selected from the group consisting of an optionally substituted alkyl group, wherein the optional substituent is selected from the group consisting of halogen, cyano, O-alkyl (preferably the optional substituent is halogen, more preferably F or CI), and aryl which is optionally substituted by halogen (preferably F or CI), cyano, alkyl or CF₃ (preferably the optional substituent is halogen, more preferably F or CI).

In a preferred embodiment, R¹⁰ is independently for each occurrence selected from the group consisting of a tertiary butyl group or isopropyl group, which is optionally substituted by a substituent which is selected from the group consisting of halogen, cyano, alkyl, O-alkyl which is optionally substituted by halogen (preferably F or CI), and aryl which is optionally substituted by halogen (preferably F or CI), cyano, alkyl or CF₃ (preferably the optional substituent is halogen, more preferably F or CI). More preferably the optional substituent is selected from the group consisting of halogen (preferably F), C₁₋₄ alkyl which is optionally substituted by halogen (preferably F), O-C₁₋₄ alkyl which is optionally substituted by halogen (preferably F) and phenyl which is optionally substituted by halogen (preferably F).

Specific examples of Al(OR¹⁰)₄ and (OR¹⁰)₃Al-F-Al(OR¹⁰)₃ include, but are not limited to, Al(OC(H)(CF₃)₂)₄, Al(OC(CH₃)(CF₃)₂)₄, Al(OC(OCH₃)(CF₃)₂)₄, Al(OC(C(CH₃)₃)(CF₃)₂)₄, Al(OC(C₆H₆)(CF₃)₂)₄, Al(OC(OCH₃)(CF₃)₂)₄, Al(OC(CF₃)₃)₄, (OC(CF₃)₃)₃Al-F-Al(OC(CF₃)₃)₃. Preferably, the aluminate anion is Al(OC(CF₃)₃)₄.

In the formula **R¹¹-[CB₁₁]R¹²₁₁** R¹¹ is selected from the group consisting of H, an aryl group and an alkyl group. Preferably, R¹¹ is selected from the group consisting of H and an alkyl group, more preferably R¹¹ is selected from the group consisting of H and a C₁₋₄ alkyl group. R¹² is independently for each occurrence selected from the group consisting of H, halogen and an alkyl group, which is optionally substituted by halogen (preferably F or CI). Preferably, R¹² is independently for each occurrence selected from the group consisting of halogen and an alkyl group, which is optionally substituted by halogen (preferably F or CI).

Specific examples of R¹¹-[CB₁₁]R¹²₁₁ include, but are not limited to, H-[CB₁₁](CH₃)₅Cl₆, C₂H₅-[CB₁₁]F₁₁, H-[CB₁₁](CF₃)₁₁. Preferably, the carborane anion is C₂H₅-[CB₁₁]F₁₁.

In the method of the present invention a single salt having the formula (IIIa) or a mixture of salts having the formula (IIIa) can be employed.

The salt having the formula (IIIa) is thought to serve as a template for the formation of the compound having the formula (IV) and the compound having the formula (V), respectively. Although the reasons are not fully understood, the choice of the anion Y¹ seems to be decisive for the formation of the desired compounds. If a related salt is employed which does not have a non-coordinating anion (NCA), e.g., a tetraalkylborate anion, the compound having the formula (IV) and the compound having the formula (V), respectively, are not formed.

### Reaction conditions

The compound having the formula (I) is reacted with the compound having the formula (II) in the presence of at least one salt having the formula (IIIa). The reaction conditions are not particularly limited but the reaction is typically conducted in the presence of an acid. The choice of acid is not particularly limited. The acid is preferably a non-aqueous, Brønsted acid or Lewis acid with a pKa of 8 or less. Examples of the acid include anhydrous hydrogen halides (HX), carboxylic acids, sulfonic acids (RSO₃H) and metal halogen Lewis acids. Preferably the acid is anhydrous HCl, benzoic acid, citric acid, formic acid, acetic acid, oxalic acid, camphor sulfonic acid, para-toluenesulfonic acid, methanesulfonic acid, trifluormethanesulfonic acid, trifluoroacetic acid (TFA), HBF₄, HPF₆, zinc(II) chloride, aluminium(III) chloride and trifluoroborate etherate. More preferably, the acid is TFA or methanesulfonic acid and even more preferably the acid is TFA. The concentration of the acid is typically in the range of about 0.001% to about 1000 % by mole of the mole of the salt of formula (IIIa), preferably in the range of about 1% to about 30% by mole of the mole of the salt of formula (IIIa).

Although the complete amount of acid can be added at the beginning of the reaction, it is preferable to add the acid continuously or in incremental amounts. It has been surprisingly found that if the acid is added continuously or in incremental amounts, then it is possible to substantially increase the yield of the desired products. Preferably the acid is added in 1/n parts of the total amount, wherein n is from 2 to 100, more preferably n is 3 to 8, even more n is 3 to 7.

The points of time when the increments are added is not particularly limited and will depend on the reaction rate, the course of the reaction and the rate with which the acid is deactivated. Typically, a skilled person can determine the duration until completion of the reaction, divide the complete reaction time by n and add the acid in 1/n parts.

The relative amounts of the compound having the formula (I), the compound having the formula (II) and the salt having the formula (IIIa) are not particularly limited. However, they will typically be used in approximately equimolar amounts (2 equivalents of compound having the formula (II), 3 equivalents of compound having the formula (I) and 1 equivalent of salt having the formula (IIIa)). In the present application, the term "approximately equimolar amounts" means that deviations of up to +/- 50% from the relative amounts specified above are tolerated.

The complete reaction time will depend, for instance, on the starting materials chosen, the amount of acid, the reaction temperature, the amount and type of molecular sieves, etc. and can be chosen appropriately by a person skilled in the art. If the desired product is a NCA complex having the formula (IV), then the reaction time is typically in the range of about 1 day to about 30 days, more typically about 4 days to about 7 days. If the desired product is a NCA complex having the formula (V), then the reaction time is typically in the range of about 1 day to about 15 days, more typically about 2 days to about 4 days. The amount of NCA complex having the formula (IV) and of NCA complex having the formula (V) in the reaction mixture can be determined by analyzing samples of the reaction mixture at regular intervals during the duration of the reaction, for instance, using ¹H NMR spectroscopy.

The complex having the formula (V) is typically formed rather cleanly before the complex having the formula (IV) is formed as the ultimate reaction product. In this case, it is sufficient to remove solvent under reduced pressure to obtain the complex having the formula (V). Depending on the starting materials chosen and the type of acid used, however, complexes having the formulae (V) and (IV) can exist as admixtures. In that case, it is possible to obtain purified complexes the formulae (V) and (IV) by crystallization.

The temperature at which the compound having the formula (I) and the compound having the formula (II) are reacted is not particularly limited and is typically in the range of about -50 °C to about 100 °C, whereby the solvent is present in liquid form. Preferably the temperature is in the range of about 15 °C to about 25 °C.

The reaction is typically conducted in an aprotic solvent, which can also be used in their deuterated form to facilitate analysis of the reaction progress. Examples of the polar aprotic solvent include, but are not limited to, chlorohydrocarbons, ether solvents (such as tetrahydrofuran, dioxane, diethyl ether), aromatic solvents (such as benzene, toluene, xylenes, hexafluorobenzene, chlororbenzene, dichlorobenzene), more preferably the polar aprotic solvent is a chlorohydrocarbon (such as a chlorinated C₁ or C₂ hydrocarbon, e.g., CHCl₃, CH₂Cl₂, CH₃Cl, C₂H₅Cl, C₂H₄Cl₂, C₂H₃Cl₃, C₂H₂Cl₄, C₂HCl₅; more particularly CHCl₃, CH₂Cl₂, and C₂H₄Cl₂), and even more preferably the solvent is CHCl₃ and CH₂Cl₂.

The concentration of the salt of formula (IIIa) is not particularly limited and is typically in the range of about 0.001 mol/l to about 1.0 mol/l. Preferably, the concentration is in the range of about 0.01 mol/l to about 0.1 mol/l.

The reaction of the compound having the formula (I) with the compound having the formula (II) is typically conducted under anhydrous conditions in order to avoid hydrolysis of the compound having the formula (I) to the corresponding ester and two equivalents of alcohol. In the present application, the term "anhydrous conditions" means that the amount of water contained in the reaction mixture is less than about 100 ppm. The anhydrous conditions can be achieved by judiciously drying all of the materials employed in the reaction as well as the reaction vessel.

The reaction progress is facilitated by the consecutive removal of compounds having the general formula R¹X¹H from the reaction mixture. Methods for achieving this include, but are not limited to, removal of R¹X¹H by distillation, removal of R¹X¹H by adsorption to porous materials, removal of R¹X¹H by liquid/liquid or liquid/solid phase extraction and removal of R¹X¹H by irreversible chemical conversion (such as oxidation). Preferably, a molecular sieve is used, which should preferably have been dried. The molecular sieve is not particularly limited but typically is a microporous material, e.g., having a pore size of about 30 nm to about 50nm.

After termination of the reaction, the desired product can be obtained from the reaction mixture by removing the solvent. Corresponding methods are well-known in the field and include, for example, removal of the solvent under reduced pressure and/or heating. Further purification is possible, if necessary, by crystallization (typically by adding an excess of pentanes to a concentrated solution in the reaction solvent).

The present method is particularly advantageous because it can be conducted in a one-pot reaction from readily available and cheap starting materials. Therefore, it provides a simple and cost-effective route to providing the crown ether complexes of the present invention. The method of the present invention relies on the self-assembly of the starting materials due to reversibility of orthoester exchange as well as a pronounced metal template effect. The desired products are obtained in a high yield and high purity.

### Further reactions

If desired, the cation M¹ can be exchanged by the cation M² and/or the anion Y¹ can be exchanged by the anion Y² in the NCA complex having the formula (IV) or (V). One option to effect cation and/or anion exchange is to reacted the NCA complex having the formula (IV) or (V) with a salt having the formula (IIIb)

M²Y² (IIIb)

to exchange the cation M¹ by cation M² and/or to exchange the anion Y¹ by the anion Y².
- **M**²: the cation can be any cation. If the cation M¹ is to be exchanged, M² can be any cation other than the cation M¹. The cation M² is not particularly limited and its choice will depend on the desired end use of the compounds. Examples of suitable cations include a cation (e.g., as defined above with respect to M¹), a proton, a compound comprising a protonated primary amino group, a compound comprising a protonated secondary amino group or a compound comprising a protonated guanidinium group.
Examples of suitable cations include, but are not limited to, alkali metals (e.g., Li, Na, K, Rb, Cs), alkali earth metals (e.g., Be, Mg, Ca, Sr, Ba, Ra), group 13 metals (e.g., Al, Ga, In, Tl), group 14 metals (e.g., Ge, Sn, Pb), transition metals of the groups 3 to 12 of the periodic table of elements (e.g., Sc, Y, Ti, Zr, Hf, V, Nb, Ta, Cr, Mo, W, Mn, Tc, Re, Fe, Ru, Os, Co, Rh, Ir, Ni, Pd, Pt, Cu, Ag, Au, Zn, Cd, Hg) and ammonium ions NR^{‡}₄, wherein R^{‡} is independently for each occurrence selected from the group consisting of H and alkyl. Preferably M² is selected from alkali metals, alkali earth metals, ammonium ions NR^{‡}₄, and the protonated forms of the amino groups Lys, Arg, His and Trp. More preferably M² is selected from Na, K, Ca, Li, Mg, NH₄⁺ and the protonated forms of the amino groups Lys, Arg, His and Trp.
In pharmaceutical applications the cation M² will typically be a metal cation, a proton, NH₄⁺, a compound comprising a protonated primary amino group, a compound comprising a protonated secondary amino group or a compound comprising a protonated guanidinium group which is capable of eliciting a desired pharmaceutical effect.
In diagnostic applications the cation M² will typically be a metal cation, a proton, NH₄⁺, a compound comprising a protonated primary amino group, a compound comprising a protonated secondary amino group or a compound comprising a protonated guanidinium group which is capable of being detected via the desired diagnostic method. Examples of possible diagnostic methods include imaging techniques such as NMR, computer tomography, magnetic resonance imaging, X-ray, detection of radioactive labels, detection of fluorescent, phosphorescent or near-infrared labels, SPECT, PET, and scintigraphy.
Examples of a compound comprising a protonated primary amino group, and a compound comprising a protonated secondary amino group include peptides, polypeptides, proteins and antibodies. The terms "peptides", "polypeptides", "proteins" and "antibodies" include peptides, polypeptides, proteins and antibodies which contain naturally occurring amino acids as well as those which contain non-naturally occurring amino acids. Derivatizations of the peptides, polypeptides, proteins and antibodies are well-known in the art and can also be used in the present invention.
- **Y²**: the anion can be any anion. If the anion Y¹ is to be exchanged, can be an anion other than the anion Y¹. The anion Y¹ is not particularly limited and its choice will depend on the desired end use of the compounds. Examples of suitable anions include halogen (such as F, Cl, Br, I), borate anions other than Ar¹ (such as BHal₄, B(alkyl)₄), phosphate anions (such as PO₄, HPO₄ or PF₆), halogenate ions (such as ClO₄⁻ ), sulfate and sulfite anions, nitrate and nitrite anions, carbonate anions, carboxylate anions (such as CH₃COO⁻), sulfonate anions (such as CF₃SO₃⁻), , preferably the anion is selected from gluconate, lactate, acetate, formate, pyruvate, galacturonate, chloride, nitrite, trifluoroacetate, bromide, nitrate, glutarate, succinate, carbonate, tartrate, benzoate, maleate, sulfate, fumarate, benzenesulfonate, phosphate, citrate and tosylate, more preferably the anion is selected from chloride and bromide.

Methods for cation and/or anion exchange are well-known in the field and include, for example, exposing a solution of the compound having the formula (IV) or (V) to an ion exchange resin loaded with the desired cation M² or anion Y², exposing a solution of the compound having the formula (IV) or (V) to a combination of molecular sieves and acid, and treating a solution of the compound having the formula (IV) or (V) with an excess of compound having the formula (IIIb) and subsequent isolation by reprecipitation or crystallization.

If desired, the NCA complex having the formula (IV) or (V) can be reacted with a compound having a strong association constant with cation M¹ to remove the ion pair comprising of cation M¹ and anion Y¹ from compounds having the formula (IV) or (V).

Suitable complexation agents are either neutral or ionic compounds, which bind more strongly to cation M¹ than the organic framework in the compound having the formula (IV) or (V). This process is not particularly limited and specific complexation agents with suitably large binding constants are known in the art. The complexation agents are not particularly limited, but are typically chelating agents (with two or more negatively polarized or anionic binding sites), neutral macrocyclic compounds (such as crown ethers), or neutral three-dimensional cage compunds (such as cryptands).

Specific examples of suitable complexation agents for the representative example of cation Na include ethylenediaminetetraacetic acid (EDTA), crown ether 1,4,7,10,13-pentaoxacyclopentadecane (15-crown-5), and cryptand 4,7,13,16,21-pentaoxa-1,10-diazabicyclo[8.8.5]tricosane (commercial name: Kryptofix® 221). The choice of the specific complexation agent will depend on the cation/anion pair to be removed, the preferred workup method and the desired use of the corresponding crown ether product.

Methods for removing a cation/anion pair by means of strong complexation agents are well-known in the field and include, adding an equimolar amount of the complexation agent to a solution of the compound having the formula (IV) or (V) and separating the resulting admixture by (re-)precipitation, (re-)crystallization, phase extraction or chromatography.

### Applications

It has surprisingly been found that the crown ether complexes having the formula (IV) and the corresponding crown ether complexes with other anions are stable under aqueous conditions in the absence of acid and even at high pH (such as pH 7 to 14).The crown ether complexes having the formula (IV) can hydrolyze and thus liberate the cation M¹ if acid is present in addition to water (such as pH 0 to 6). Because even mildly acidic conditions such as present in physiological conditions are sufficient, the crown ether complexes having the formula (IV) can be employed in a wide range of pharmaceutical and diagnostic applications such as the delivery of metal ions to a patient. Because the crown ether complexes having the formula (IV) disintegrate under physiological conditions, they can be used as "traceless" ion carriers, e.g., to deliver metal cations.

Consequently, the present application also relates to a pharmaceutical or diagnostic composition comprising a compound according to the present invention. The pharmaceutical or diagnostic composition can optionally further comprise a pharmaceutically or diagnostically acceptable exipient and/or carrier. The pharmaceutical or diagnostic composition can be obtained, e.g., by providing an admixture of a compound according to the present invention and a pharmaceutically or diagnostically acceptable exipient and/or carrier.

Details on possible cations and anions which can be used in pharmaceutical compositions as well as the formulation of such pharmaceutical compositions can be found in EP-A-2 332 - 929.

The compounds of the present invention can be administered to a patient in the form of a pharmaceutical composition which can optionally comprise one or more pharmaceutically or diagnostically acceptable excipient(s) and/or carrier(s).

The compounds of the present invention can be administered by various well known routes, including oral, rectal, intragastrical, intracranial and parenteral administration, e.g. intravenous, intramuscular, intranasal, intradermal, subcutaneous, and similar administration routes. Transdermal and transmucosal administration are particularly preferred. Depending on the route of administration different pharmaceutical formulations are required and some of those may require that protective coatings are applied to the drug formulation to prevent degradation of a compound of the invention in, for example, the digestive tract.

Thus, preferably, a compound of the invention is formulated as a cream, a lotion, a syrup, an infusion or injection solution, a spray, a tablet, a capsule, a caplet, lozenge, a suppository, a plaster, a band-aid, a retard capsule, a powder, or a slow release formulation.

Suitable excipients can be found, for example, in the Handbook of Pharmaceutical Excipients, published by the American Pharmaceutical Association, which is herein incorporated by reference.
It is to be understood that depending on the severity of the disorder and the particular type which is treatable with one of the compounds of the invention, as well as on the respective patient to be treated, e.g. the general health status of the patient, etc., different doses of the respective compound are required to elicit a therapeutic or prophylactic effect. The determination of the appropriate dose lies within the discretion of the attending physician. The duration of therapy with a compound of the invention will vary, depending on the severity of the disease being treated and the condition and idiosyncratic response of each individual patient.
If a person is known to be at risk of developing a disease treatable with a compound of the invention, prophylactic administration of the biologically active blood serum or the pharmaceutical composition according to the invention may be possible. In these cases the respective compound of the invention is preferably administered in above outlined preferred and particular preferred doses on a daily basis. This administration can be continued until the risk of developing the respective viral disorder has lessened. In most instances, however, a compound of the invention will be administered once a disease/disorder has been diagnosed. In these cases it is preferred that a first dose of a compound of the invention is administered one, two, three or four times daily.

If the compounds of the present invention are detectably labelled, they can be employed in the diagnosis of a disease. The formulation of the diagnostic composition will depend on the route of administration, the type of label and the disorder to be diagnosed. Suitable formulations can be determined by a person skilled in the field. The label can be present at any available position. Examples of labels include radioactive labels, fluorescent labels, spin labels and near-infrared labels.

Sodium or lithium crown ether complexes having the formula (IV) and the corresponding crown ether complexes with other anions could also be useful as electrolytes in sodium or lithium ion batteries. It is expected that the present crown ether complexes could provide higher ionic conductivity compared to the salts such as Nape or LiPF₆ which are currently used as an organic solvent in these devices.

A further area of technology in which the compounds described herein could be useful is molecular sensing. If equipped with suitable R groups (e.g. a dye or anthracene), the crown ether compounds having the formula (IV) or (V) and the corresponding crown ether complexes with other anions could be used as optical indicators in quick-tests for a wide range of cations (each cation is expected to generate a particular colour or fluorescence signal with potentially very high specificity).

The crown ether complexes having the formula (V) and the corresponding crown ether complexes with other anions and/or other cations M³ are not only useful as intermediates in the preparation of the crown ether complexes having the formula (IV). Rather they are also suitable in the same applications as the crown ether complexes having the formula (IV). Because the cation M¹ can be exchanged by a cation having a primary or secondary protonated amino group or a protonated guanidinium group it is additionally possible to use the compounds having the formula (V) and the corresponding crown ether complexes with other anions and/or other cations M³ in a wide range of pharmaceutical and diagnostic applications such as the delivery of the corresponding cations to a patient.

The crown ether complexes having the formula (IV) or (V) could also be covalently (e.g. through the group R) or non-covalently integrated into polymers.

Various modifications and variations of the invention will be apparent to those skilled in the art within the scope of the appended claims. Although the invention has been described in connection with specific preferred embodiments, it should be understood that the invention as claimed should not be unduly limited to such specific embodiments. Indeed, various modifications of the described modes for carrying out the invention which are obvious to those skilled in the relevant fields are intended to be covered by the present invention, as far as they fall under the scope of the appended claims. The following examples are merely illustrative of the present invention and should not be construed to limit the scope of the invention as indicated by the appended claims in any way.

### Examples

### Example 1: Template-synthesis of a dynamic cryptate.

Based on reports on dynamic 'scaffolding ligands' (*O*,*N*,*P*-orthoesters),^{29,30} the exchange reaction of *O*,*O*,*O*-orthoesters with simple alcohols was investigated from a DCC perspective. It was recognized from these model studies that the tripodal architecture of carboxylic orthoesters³¹ could potentially provide a unique platform for the template synthesis of an unprecedented class of constitutionally dynamic cryptates.

To test this hypothesis, a chloroform solution of two bulk chemicals, trimethyl orthoacetate (1) and diethylene glycol (2), was treated with catalyst trifluoroacetic acid (TFA) and stoichiometric metal template (Figure 2). Analysis of these initial experiments by ¹H NMR spectroscopy and ESI mass spectrometry revealed that complex mixtures, containing the desired crown ether complex among other exchange products, had formed. Careful optimization of the reaction conditions (use of molecular sieves for exclusion of moisture and removal of methanol; use of 'non-coordinating' counteranion⁴² tetrakis[3,5-bis(trifluoromethyl)phenyl]borate (BArF))³²⁻³⁵ led to the formation of the crown ether complex **Na[*o*-Me₂-1.1.1]BArF** (named in loose analogy to Lehn's classic cryptates; '*o*' stands for orthoester) as major reaction product (isolated yields: 60-70%).

As shown in the ¹H NMR spectra presented in Figure 2, the reversible reaction between **1** and **2** initially generates a remarkable diversity of different exchange products (various degrees of replacement of MeOH by **2,** as well as formation of macrocyclic and oligomeric products; Figure 2b). Slow removal of MeOH by molecular sieves (4Å) allows the system to converge to the final product (Figure 2c).

In Figure 2 the one-pot self-assembly of dynamic crown ether complex Na[*o*-Me₂-1.1.1]BArF and partial ¹H NMR spectra showing the evolution of the dynamic system over time are given.
**a**, Starting materials.
**b**, Complex mixture that forms rapidly after addition of acid catalyst (1 h).
**c**, Removal of MeOH leads to formation of **Na[*o*-Me₂-1.1.1]BArF** as major product (5 d), alongside hydrolysis product MeOAc (singlets at 3.6 and 2.0 ppm).
**d**, Removal of solvent under reduced pressure provides essentially pure material.

Reaction conditions: trimethylorthoacetate (120 µmol), diethylene glycol (180 µmol), NaBArF (60 µmol), TFA (3.0 µmol), molecular sieves (4 Å, 1 g), CDCl₃ (6.0 mL), 5 d, room temperature (RT).

It should be noted that during the exchange process, water should be excluded from the reaction mixture, which is not trivial to achieve, because even rigorously dried molecular sieves tend to slowly release residual water. As a consequence, hydrolysis of **1** can lead to the slow formation of methyl acetate (MeOAc) as a side product (see Figure 2c). From a preparative standpoint, the formation of MeOAc is not a problem, however, because it can easily be removed under reduced pressure (see Figure 2d). Also, its formation as an exclusive side product provides two valuable pieces of information on the dynamic system under study. First, the partial decomposition of orthoester **1** implies that the ratio of orthoester to diol is not at the ideal initial value of 2:3. Nevertheless, it was found that the crown ether complex as a major product even in experiments where the effective stoichiometry was far from ideal, indicating that there is a strong thermodynamic bias for the formation of the final crown ether complex, at least in the presence of sodium template. Second, the fact that only MeOAc is generated by hydrolysis during the exchange reaction suggests that all exchange products incorporating at least one diethylene glycol moiety (generated much more rapidly than MeOAc) are kinetically stabilized against hydrolysis, presumably due to (chelate) binding of the sodium ion.

This kinetic stabilization by metal binding, which was previously observed and systematically studied in acetal crown ethers, ³⁶⁻³⁸ is remarkably pronounced in isolated crown ether complex **Na[*o*-Me₂-1.1.1]BArF.** In a water-saturated chloroform solution of pristine **Na[*o*-Me₂-1.1.1]BArF** and trimethyl orthoacetate (**1**), for example, the presence of water resulted in complete hydrolysis of **1**, while crown ether complex **Na[*o*-Me₂-1.1.1]BArF** remained stable. In the absence of acid, **Na[*o*-Me₂-1.1.1]BArF** is in fact stable enough to be analyzed by reverse-phase HPLC-MS (in H₂O/MeOH) and can be passed over a plug of conventional silica gel without noticeable decomposition. Both observations are highly unusual for *O*,*O*,*O*-orthoesters that are not stabilized by the presence of 5- or 6-membered rings. Due to these extraordinary properties, it is believed that orthoester cages such as **Na[*o*-Me₂-1.1.1]BArF** could be useful for the traceless delivery of cationic molecular guests into biological systems (**Na[*o*-Me₂-1.1.1]BArF** hydrolyzes readily in the presence of excess water and acid).

### Example 2: Solid state structure and stability against water.

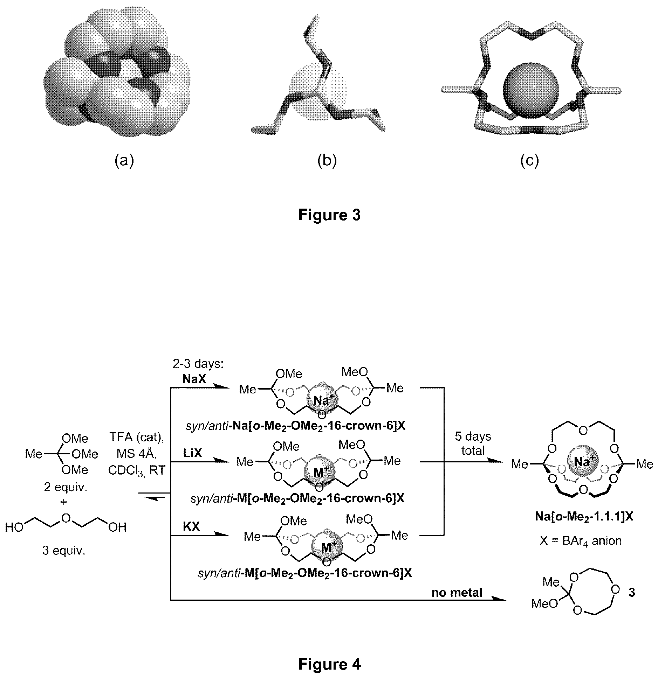

Single crystals of **Na[*o*-Me₂-1.1.1]BArF** suitable for X-ray crystallography were obtained by slow diffusion of cyclopentane into dichloromethane. The solid state structure (Figure 3) shows that the sodium ion is bound to all nine surrounding oxygen atoms with Na-O bond lengths (mean 2.56 Å) essentially identical those found in Lehn's classic cryptate Na[2.2.2] (mean 2.57 Å).³⁹ The relatively small distances between the three diethylene glycol chains (interchain central O-O distance: 4.5 Å; interchain central C-O distance: 4.9 Å; see space-filling model in Figure 3) and the rigid architecture of the cage (no inversion possible at the orthoester carbon centre) suggest that the metal ion is tightly bound within the cavity of the cage.⁴⁰

Figure 3 shows the solid state structure of **Na[*o*-Me₂-1.1.1]BArF.** Determined by single-crystal X-ray diffraction, crystals were obtained by slow diffusion of cyclopentane into dichloromethane. Counteranion, hydrogen atoms and disorder (50% occupancy was observed along the CH₂-CH₂-O-CH₂-CH₂ chains of the complex) are omitted for clarity. Oxygen atoms are shown in red, carbon atoms in gray. The sodium ion is shown at 70% (stick model) and 100% (space-filling model) of van der Waals radius. Na-O bond lengths: 2.51-2.58 Å (six orthoester oxygens), 2.55-2.62 Å (three chain oxygens).

### Example 3: Reaction outcome with other metal templates: crown ether complexes having the formula (V) and leaching of sodium from 4 A molecular sieves.

To confirm that a metal template effect is indeed responsible for the remarkably clean formation of **Na[*o*-Me₂-1.1.1]BArF,** the exchange reaction between **1** and **2** in the absence of template and in the presence of other metal templates was studied under otherwise identical reaction conditions. As shown in Figure 4, the exchange reaction without template mainly gave rise to exchange product **4,** in which one molecule of diethylene glycol (**2**) has displaced two molecules of methanol. Theoretically, it should be possible to remove the last equivalent of methanol by increasing the time during which the mixture is exposed to molecular sieves, but it was found that the system appears to have a very strong tendency to remain at this particular state (i.e. product **3**). This finding suggests that in the absence of a template, molecular sieves are not providing a strong enough driving force for further shifting the equilibrium distribution towards other possible exchange products.

Using metal templates lithium tetrakis(pentafluorophenyl)borate (LiTPFPB) and potassium tetrakis[3,5-bis(trifluoromethyl)phenyl]borate (KBArF), it was noticed that after 2 to 3 days reaction time a pair of altogether different reaction products had formed. Isolation of the corresponding material and careful analysis of 1D and 2D NMR spectra revealed that unprecedented crown ethers¹ **(*o*-Me₂-(OMe)₂-16-crown-6)** and **(*o*-Me₂-(OMe)₂-16-crown-6)** had formed as a mixture of syn and anti diastereomers (see ¹H NMR spectra and chemical structures in Figure 4). Further experiments revealed that the crown ether **Na(*o*-Me₂-(OMe)₂-16-crown-6)⁺** and orthoformate crown ethers **(*o*-H₂-(OMe)₂-16-crown-6)** could also be obtained as predominant exchange products once the exposure to molecular sieves had led to the removal of precisely two equivalents of methanol (2 to 3 days reaction time). The dynamic chemical system is thus not only responsive to the described metal template effect, but also to the precise quantity of available methanol. As a result, molecular sieves can be seen as an entropic auxiliary for achieving the otherwise highly unlikely self-assembly of two orthoesters, three diols and one metal ion.

Upon exposure to molecular sieves and acid catalyst, the crown ethers originating from salts LiTPFPB and KBArF could be further transformed into compounds whose NMR spectra closely resembled those of **Na[*o*-Me₂-1.1.1]BArF.** Mass spectrometry, ²³Na NMR spectroscopy and atom absorption spectroscopy (AAS) provided conclusive evidence that these cage compounds are in fact sodium cryptates **Na[*o*-Me₂-1.1.1]⁺.** Based on AAS and ²³Na NMR spectroscopy, it is assumed that this unexpected finding can be traced back to a relatively slow ion exchange that occurs between Li or K ions in solution and Na ions embedded in the crystal lattice of 4 Å molecular sieves. Interestingly, there are literature reports on this type of ion exchange in strongly acidic aqueous medium.⁴³ The remarkable preference for cryptate **Na[*o*-Me₂-1.1.1]⁺** over the potential lithium or potassium cryptates is most likely a result of the differences in ionic radius between lithium (0.9 Å), sodium (1.2 Å) and potassium (1.6 Å).⁴⁰ At least when HO-(CH₂)₂-O-(CH₂)₂-OH is used as a compound having formula (II), only sodium appears to have the right size for forming nine efficient metal-oxygen bonds, while not inducing energetically costly conformations within the organic host. It is reasonable to expect that other combinations of compounds having formula (II) will allow the incorporation of other cations.

It can be seen that a dynamic system based on two strikingly simple organic starting materials converges to three distinct exchange products under the influence of dry molecular sieves: (i) in the absence of a template effect, a simple exchange product featuring an 8-membered ring is formed. (ii) In the presence of a sodium template, which can also be generated via cation exchange with molecular sieves, an unprecedented dynamic crown ether complexes having the formula (IV) is formed. (iii) In the presence of suitable lithium, sodium or potassium templates, dynamic crown ether complexes having the formula (V) can be isolated as intermediates en route to the formation of the sodium crown ether complex having the formula (V). The sodium crown ether complex **Na[*o*-Me₂-1.1.1]BArF** has been characterized, which was found to be surprisingly stable against water in neutral solution. The solid state structure of **Na[*o*-Me₂-1.1.1]BArF** and competition experiments in solution suggest that the encapsulated metal ion is kinetically trapped within the host as long as orthoester exchange is not switched on. It is believe that this property could make orthoester cages useful for the traceless delivery of cations into biological systems.⁴¹ Besides their interesting dynamic properties, orthoester cryptates offer preparative advantages in comparison to their classic analogues: their synthesis relies on a one-pot, six-component ring-closing reaction, and substituted cages **([*o*-R₂-1.1.1])** are accessible simply by using different orthoesters as starting materials.

### General Experimental Section

### Reagents and instruments

All commercially purchased reagents were used without further purification. Molecular sieves were dried for 3 days at 150 °C under reduced pressure (8.0 x 10⁻³ mbar) before use. CDCl₃ (99.8%, Deutero GmbH), DMSO-d₆, MeCN-d₃ and benzene-d₆ were stored over 4Å molecular sieves. Methanol and ethanol were stored over 3Å molecular sieves. NMR spectra were recorded on a Bruker Avance 400 (¹H: 400 MHz and ¹³C: 100 MHz) spectrometer at 298 K. NMR spectra were referenced to the residual solvent peak (¹H: CDCl₃, 7.24 ppm; ¹³C: CDCl₃, 77.23 ppm). Coupling constants (J) are denoted in Hz and chemical shifts (δ) in ppm. Mass spectra were obtained on Bruker micro TOF II and Bruker Maxis 4G (ESI⁺, Toluene/Acetonitrile) instruments. HPLC-MS analysis was performed on a Shimadzu LCMS 2020 instrument (column: Kinetex C18, 2.6 µm, 100 x 4.6 mm; mobile phase: H₂O/MeOH 80% → 100 % MeOH; flow rate: 0.3 mL/min).

### Preparation of stock solutions

To achieve a high level of stoichiometric accuracy, metal salts and diethylene glycol were added from stock solutions that were dried over molecular sieves before orthoester and acid catalyst were added. In a typical example, sodium tetrakis[3,5-bis(trifluoromethyl)phenyl]-borate (NaBArF, 0.14 mmol, 127.8 mg) and diethylene glycol (DEG, 0.42 mmol, 39.9 µL) were dissolved in CDCl₃ (14 mL) and dried with 4 Å molecular sieves (1 g) for 3 days. From this stock solution 6 mL were added to the reaction vessel.

To obtain the acid stock solution, trifluoroacetic acid (TFA, 240 µmol, 18.4 µL) was topped up with CDCl₃ to obtain a total volume of 2 mL. Of this stock solution, 50 µL were added to the reaction vessel.

### Exclusion of atmospheric moisture

All solvents were dried over molecular sieves for at least 24 hours. Most reactions were performed in oven-dried, screw-capped scintillation vials. All reactions were carried out under a "non-rigorous" nitrogen atmosphere (no degassing of the solvent necessary, but dried molecular sieves and closed reaction vessels should be used).

Rigorous exclusion of moisture is only necessary during the exchange process (when acid catalyst is present). After the acid is quenched, orthoesters can be surprisingly stable.

### Synthetic procedures and characterization data

### Synthesis of Na[o-Me₂-1.1.1]BArF

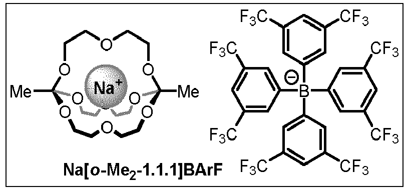

To 6 mL of the NaBArF stock solution were added 4 Å molecular sieves (1 g) and the reaction mixture was left standing at room temperature. After 16 h, 5 mol% TFA (50 µL) was added from a stock solution, the mixture was shaken and trimethyl orthoacetate (0.12 mmol, 15.4 µL) was added. Every 24 h, 5 mol% TFA was added to keep the exchange reaction active (molecular sieves slowly transform the acid catalyst into inactive anhydride and/or esters). The reaction progress was monitored regularly by ¹H NMR spectroscopy. After 5 days, the solvent was removed under reduced pressure and the title compound was obtained in 67% yield.
¹H NMR (400 MHz, CDCl₃): δ = 7.68 (t, *J* = 2.8 Hz, 8H), 7.51 (s, 4H), 3.79 - 3.77 (m, 12H), 3.50 - 3.48 (m, 12H), 1.43 ppm (s, 6H).
¹³C NMR (100 MHz, CDCl₃): δ = 162.8, 162.3, 161.8, 161.3, 135.1, 129.7, 129.4, 129.1, 128.9, 128.8, 126.2, 123.5, 120.8, 117.7, 113.0, 69.2, 62.0, 17.7 ppm.
¹¹B NMR (128 MHz, CDCl₃): δ = 6.7 ppm.
¹⁹F NMR (282 MHz, CDCl₃): δ = -62.1 ppm.
HRMS (ESI⁺): *m*/*z* = 389.1794 [M+Na]⁺ (calcd. 389.1782 for C₁₆H₃₀O₉Na).

### Synthesis of Na[o-Et₂-1.1.1]BArF

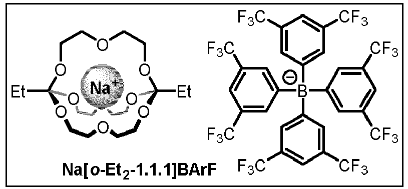

To 6 mL of the NaBArF stock solution were added 4 Å molecular sieves (1 g) and the reaction mixture was left standing at room temperature. After 16 h, 1 mol% TFA (10 µL) was added from a stock solution, the mixture was shaken and trimethyl orthopropionate (0.12 mmol, 16.5 µL) was added. Every 24 hours, 1 mol% TFA was added to keep the exchange reaction active (molecular sieves slowly transform the acid catalyst into inactive anhydride and/or esters). The reaction progress was monitored regularly by ¹H NMR spectroscopy. After 5 days, the solvent was removed under reduced pressure and the title compound was obtained in 50% yield.
¹H NMR (400 MHz, CDCl₃): δ = 7.68 (t, *J* = 2.8 Hz, 8H), 7.51 (s, 4H), 3.99 - 3.68 (m, 12H), 3.64 - 3.35 (m, 12H), 1.85 (q, *J* = 7.5 Hz, 4H), 0.94 (t, *J* = 7.5 Hz, 6H).
¹³C NMR (100 MHz, CDCl₃): δ = 162.8, 162.3, 161.8, 161.3, 135.1, 129.7, 129.4, 129.1, 128.9, 128.8, 126.2, 123.5, 120.8, 117.7, 114.3, 69.7, 62.6, 27.2, 7.7.
HRMS (ESI⁺): *m*/*z* = 417.2091 [M+Na]⁺ (calcd. 417.2095 for C₁₈H₃₄O₉Na).

### Synthesis of Na[o-Me₂-1.1.1]TCPB

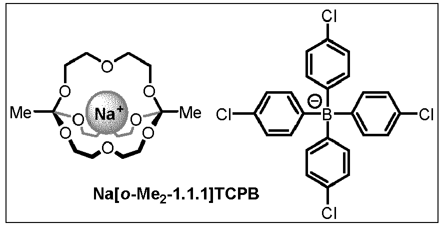

Potassium tetrakis(4-chlorophenyl)borate (KTCPB) and 4 Å molecular sieves (1 g) were added to a solution of diethylene glycol (0.18 mmol, 17.1 µL) in CDCl₃ and the reaction mixture was left standing at room temperature. After 16 h, 1 mol% TFA (10 µL) was added from a stock solution, the mixture was shaken and trimethyl orthoacetate (0.12 mmol, 15.4 µL) was added. Every 24 hours, 1 mol% TFA was added to keep the exchange reaction active (molecular sieves slowly transform the acid catalyst into inactive anhydride and/or esters). The reaction progress was monitored regularly by ¹H NMR spectroscopy. After 3 days, the solvent was removed under reduced pressure and the title compound was obtained in 60% yield.
¹H NMR (400 MHz, CDCl₃): δ = 7.54 (d, *J* = 8.4 Hz, 2H), 7.46 (d, *J* = 8.8 Hz , 2H), 7.00 (d, *J* = 8.0 Hz , 12H), 3.78 - 3.76 (m, 12H), 3.49 - 3.47 (m, 12H), 1.44 (s, 6H).
¹³C NMR (100 MHz, CDCl₃): δ = 161.8, 161.4, 160.9, 160.4, 137.5, 128.1, 126.0, 113.0, 69.2, 62.1, 18.0.
HRMS (ESI⁺): *m*/*z* = 389.1809 [M+Na]⁺ (calcd. 389.1782 for C₁₆H₃₀O₉Na).

### Synthesis of Na[o-H₂-(OMe)₂-16-crown-6]BArF (syn & anti)

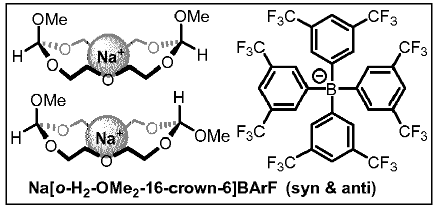

To 6 mL of the NaBArF stock solution were added 4 Å molecular sieves (1 g) and the reaction mixture was left standing at room temperature. After 16 h, 1 mol% TFA (50 µL) was added from a stock solution, the mixture was shaken and trimethyl orthoacetate (0.12 mmol, 15.4 µL) was added. Every 24 hours, 1 mol% TFA was added to keep the exchange reaction active (molecular sieves slowly transform the acid catalyst into inactive anhydride and/or esters). The reaction progress was monitored regularly by ¹H NMR spectroscopy. After 2 days, the solvent was removed under reduced pressure and the title compound was obtained as a mixture of syn and anti isomers in 90% yield.
¹H NMR (400 MHz, CDCl₃): δ = 7.67 (t, *J* = 2.8 Hz, 16H), 7.52 (s, 8H), 5.09 (s, 2H), 5.06 (s, 2H), 3.87 - 3.81 (m, 8H), 3.62 - 3.52 (m, 24H), 3.31 (s, 6H), 3.30 (s, 6H).
¹³C NMR (100 MHz, CDCl₃): δ = 162.6, 162.1, 161.6, 161.1, 135.0, 129.6, 129.3, 129.0, 128.8, 128.7, 126.1, 123.4, 120.7, 117.8, 113.6, 113.0, 77.4, 70.7, 69.2, 69.1, 64.5, 64,4, 63.6, 61.3, 52.7.
HRMS (ESI): *m*/*z* = 319.1366 [M+Na]⁺ (calcd. 319.1363 for C₁₂H₂₄O₈Na).

### Synthesis of M[o-H₂-(OMe)₂-16-crown-6]TPFPB

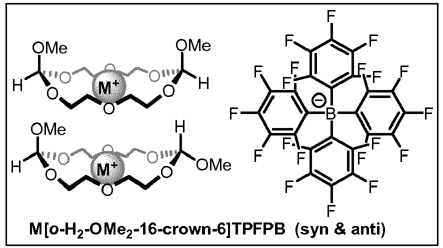

Potassium tetrakis(pentafluorophenyl)borate (KTPFPB) and 4 Å molecular sieves (1 g) were added to a solution of diethylene glycol (0.18 mmol, 17.1 µL) in CDCl₃ and the reaction mixture was left standing at room temperature. After 16 h, 1 mol% TFA (10 µL) was added from a stock solution, the mixture was shaken and trimethyl orthoformate (0.12 mmol, 15.4 µL) was added. Every 24 hours, 1 mol% TFA was added to keep the exchange reaction active (molecular sieves slowly transform the acid catalyst into inactive anhydride and/or esters). The reaction progress was monitored regularly by ¹H NMR spectroscopy. After 8 days, the solvent was removed under reduced pressure and the title compound was obtained in 90% yield.
¹H NMR (400 MHz, CDCl₃): δ = 5.15 (s, 2H), 5.14 (s, 2H), 3.93 - 3.88 (m, 8H), 3.66 - 3.59 (m, 24H), 3.36 (s, 6H), 3.35 (s, 6H).
HRMS (ESI): *m*/*z* = 335.1097 [M+K]⁺ (calcd. 335.1103 for C₁₂H₂₄O₈K).

### Synthesis of Na[o-Me₂-(OMe)₂-16-crown-6]BArF

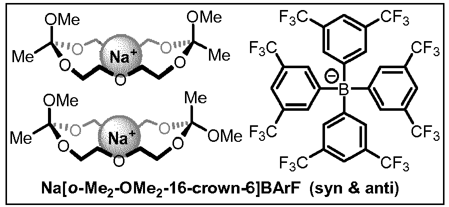

To 6 mL of the NaBArF stock solution were added 4 Å molecular sieves (1 g) and the reaction mixture was left standing at room temperature. After 16 h, 5 mol% TFA (50 µL) was added from a stock solution, the mixture was shaken and trimethyl orthoacetate (0.12 mmol, 15.4 µL) was added. Every 24 hours, 5 mol% TFA was added to keep the exchange reaction active (molecular sieves slowly transform the acid catalyst into inactive anhydride and/or esters). The reaction progress was monitored regularly by ¹H NMR spectroscopy. After 3 days, the solvent was removed under reduced pressure and the title compound was obtained as a mixture of syn and anti isomers in 70% yield.
¹H NMR (400 MHz, CDCl₃): δ = 7.67 (t, *J* = 2.8 Hz,8H), 7.52 (s, 4H), 3.81 - 3.76 (m, 12H), 3.58 - 3.48 (m, 12H), 3.26 (s, 3H), 3.24 (s, 3H), 1.44 (s, 3H), 1.43 (s, 3H).

### Synthesis of M[o-Me₂-(OMe)₂-16-crown-6]TPFPB

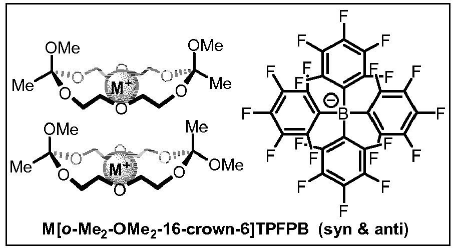

To 6 mL of the lithium tetrakis(pentafluorophenyl)borate ethyl etherate (LiTPFPB) stock solution were added 4 Å molecular sieves (1 g) and the reaction mixture was left standing at room temperature. After 16 h, 5 mol% TFA (10 µL) was added from a stock solution, the mixture was shaken and trimethyl orthoacetate (0.12 mmol, 15.4 µL) was added. Every 24 hours, 5 mol% TFA was added to keep the exchange reaction active (molecular sieves slowly transform the acid catalyst into inactive anhydride and/or esters). The reaction progress was monitored regularly by ¹H NMR spectroscopy. After 5 days, the solvent was removed under reduced pressure and the title compound was obtained as a mixture of syn and anti isomers in 60% yield.
¹H NMR (400 MHz, CDCl₃): δ = 3.88 - 3.81 (m, 4H), 3.64 - 3.56 (m, 12H), 3.28 (s, 3H), 3.21 **(s, 3H), 1.47 (s, 3H), 1.40 (s, 3H).**

### Comparative example 1 (salt NaCl)

To 6 mL of a stock solution/suspension of NaCl were added 4 Å molecular sieves (1 g) and the reaction mixture was left standing at room temperature. After 16 h, 1 mol% TFA (50 µL) was added from a stock solution, the mixture was shaken and trimethyl orthoacetate (0.12 mmol, 15.4 µL) was added. Every 24 h, 1 mol% TFA was added. The reaction progress was monitored regularly by ¹H NMR spectroscopy. After 7 days, neither product having the formula (V) nor product having the formula (IV) was observed by ¹H NMR spectroscopy.

### Comparative example 2 (salt NaPF₆)

To 6 mL of a stock solution/suspension of NaPF₆ in CDCl₃ were added 4 Å molecular sieves (1 g) and the reaction mixture was left standing at room temperature. After 16 h, 1 mol% TFA (50 µL) was added from a stock solution, the mixture was shaken and trimethyl orthoacetate (0.12 mmol, 15.4 µL) was added. Every 24 h, 1 mol% TFA was added. The reaction progress was monitored regularly by ¹H NMR spectroscopy. After 7 days, neither product having the formula (V) nor product having the formula (IV) was observed by ¹H NMR spectroscopy.

### Comparative example 3 (salt NaBF₄)

To 6 mL of a stock solution/suspension of NaBF₄ in CDCl₃ were added 4 Å molecular sieves (1 g) and the reaction mixture was left standing at room temperature. After 16 h, 1 mol% TFA (50 µL) was added from a stock solution, the mixture was shaken and trimethyl orthoacetate (0.12 mmol, 15.4 µL) was added. Every 24 h, 1 mol% TFA was added. The reaction progress was monitored regularly by ¹H NMR spectroscopy. After 7 days, neither product having the formula (V) nor product having the formula (IV) was observed by ¹H NMR spectroscopy.

### Comparative example 4 (salt NaBEt₄)

To 6 mL of a stock solution/suspension of NaBEt₄ in CDCl₃ were added 4 Å molecular sieves (1 g) and the reaction mixture was left standing at room temperature. After 16 h, 1 mol% TFA (50 µL) was added from a stock solution, the mixture was shaken and trimethyl orthoacetate (0.12 mmol, 15.4 µL) was added. Every 24 h, 1 mol% TFA was added. The reaction progress was monitored regularly by ¹H NMR spectroscopy. After 7 days, neither product having the formula (V) nor product having the formula (IV) was observed by ¹H NMR spectroscopy.

### Crystallographic data

### Compound Na[o-Me₂-1.1.1]BArF:

**Na[*o*-Me₂-1.1.1]BArF** was crystallized by slow diffusion of cyclopentane into a solution of **Na[*o*-Me₂-1.1.1]BArF** in dichloromethane.

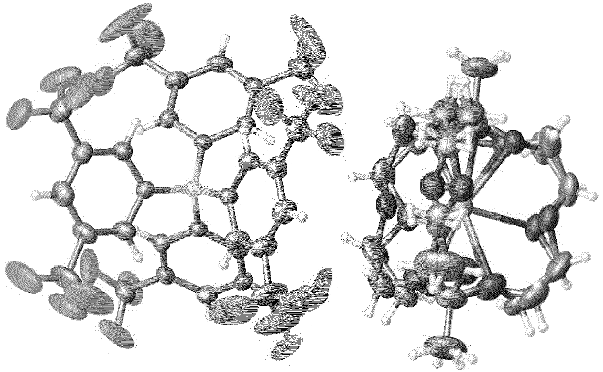

### Notes on structure solution and refinement for Na[o-Me₂-1.1.1]BArF:

| | |
|---|---|
| Goniometer type: | Agilent SuperNova with Atlas detector |
| Structure solution : | SHELXS-2013 |
| Structure refinement: | SHELXL-2013 |
| Hydrogen treatment: | riding model |
| **Disorder:** | **Cage: 50:50 % disorder of the three aliphatic chains** |
| | CF₃-Groups also show some slight disorder, but could not be resolved because of lack of sufficient data intensities |
| Comments: | Asym. unit contains some disordered solvent molecule (dichloromethane), which could not be resolved, and therefore was masked during refinement |

### Crystal data for Na[o-Me₂-1.1.1]BArF:

| | |
|---|---|
| Identification code | rb01_3 |
| Empirical formula | C₄₈H₄₂BF₂₄NaO₉ |
| Formula weight | 1252.61 |
| Temperature/K | 173.00(10) |
| Crystal system | monoclinic |
| Space group | C2/m |
| a/Å | 18.4687(4) |
| b/Å | 16.8775(3) |
| c/Å | 18.8277(4) |
| α/° | 90 |
| β/° | 107.326(2) |
| γ/° | 90 |
| Volume/Å³ | 5602.4(2) |
| Z | 4 |
| ρ_{calc}mg/mm³ | 1.485 |
| µ/mm⁻¹ | 1.443 |
| F(000) | 2536.0 |
| Crystal size/mm³ | 0.1927 × 0.1091 × 0.0801 |
| 20 range for data collection | 7.26 to 122.92° |
| Index ranges | -20 ≤ h ≤ 19, -18 ≤ k ≤ 18, -12 ≤ l ≤ 21 |
| Reflections collected | 16070 |
| Independent reflections | 4452[R(int) = 0.0405] |
| Data/restraints/parameters | 4452/16/432 |
| Goodness-of-fit on F² | 1.100 |
| Final R indexes [I>=2σ (I)] | R₁ = 0.0712, wR₂ = 0.2006 |
| Final R indexes [all data] | R₁ = 0.0821, wR₂ = 0.2128 |
| Largest diff. peak/hole / e Å⁻³ | 0.74/-0.42 |

## Claims

1. A method comprising the step of reacting
at least one compound having the formula (I) 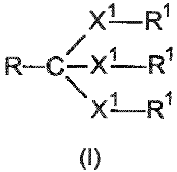 wherein
**X¹** is independently for each occurrence selected from the group consisting of 0 and S;
**R¹** is independently for each occurrence selected from the group consisting of alkyl and carbocyclyl; and
**R** is H or an organic moiety;
with at least one compound having the formula (II) 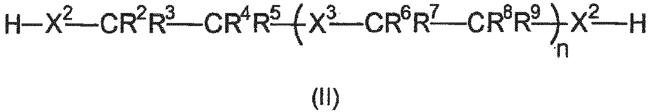 wherein
**X²** is independently for each occurrence selected from the group consisting of O and S;
**X³** is independently for each occurrence selected from the group consisting of O and S;
**R²**, **R³**, **R⁴**, **R⁵**, **R⁶**, **R⁷**, **R⁸**, and **R⁹** are independently for each occurrence selected from the group consisting of H, optionally substituted carbocyclyl and optionally substituted alkyl or any two of R², R³, R⁴, R⁵, R⁶, R⁷, R⁸, and R⁹ which are attached to the same carbon atom or two adjacent carbon atoms can be bonded to form a 3- to 7-membered carbocyclic ring;
**n** is independently for each occurrence selected from the group consisting of 0,1, 2 and 3;
in the presence of at least one salt having the formula (IIIa)
M¹Y¹ (IIIa)
wherein
**M¹** is a cation; and
**Y¹** is a non-coordinating anion (NCA) selected from the group consisting of tetraarylborate anions B(Ar¹)₄, aluminate anions Al(OR¹⁰)₄ or (R¹⁰O)₃Al-F-Al(OR¹⁰)₃ and carborane anions R¹¹-CB₁₁R¹²₁₁;
wherein in the formula **B(Ar¹)**₄Ar¹ is independently for each occurrence selected from the group consisting of an optionally substituted aryl or optionally substituted heteroaryl group;
wherein in the formulae **Al(OR¹⁰)₄** or **(R¹⁰O)₃Al-F-Al(OR¹⁰)₃** R¹⁰ is independently for each occurrence selected from the group consisting of an optionally substituted alkyl group, wherein the optional substituent is selected from the group consisting of halogen, cyano, O-alkyl, and aryl which is optionally substituted by halogen, cyano, alkyl or CF₃;
wherein in the formula **R¹¹-[CB₁₁]R¹²₁₁**R¹¹ is selected from the group consisting of H, an aryl group and an alkyl group and R¹² is independently for each occurrence selected from the group consisting of H, halogen and an alkyl group, which is optionally substituted by halogen;
to form a NCA complex having the formula (IV)

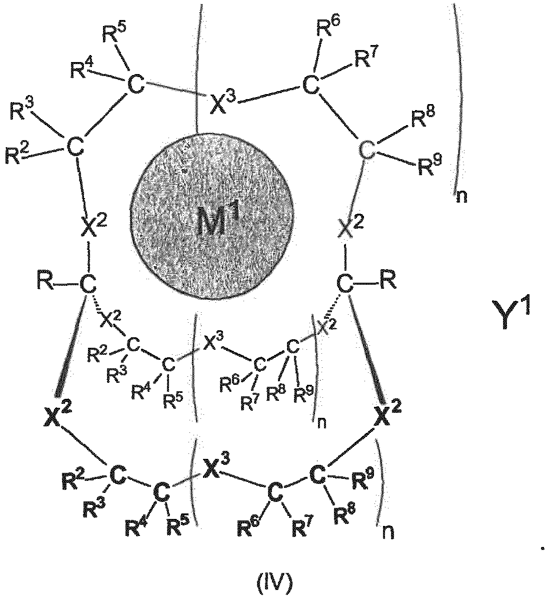

2. A method comprising the step of reacting
at least one compound having the formula (I) 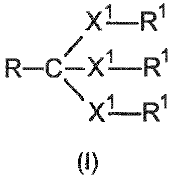 wherein
**X¹** is independently for each occurrence selected from the group consisting of O and S;
**R¹** is independently for each occurrence selected from the group consisting of alkyl, and carbocyclyl; and
**R** is H or an organic moiety;
with at least one compound having the formula (II) 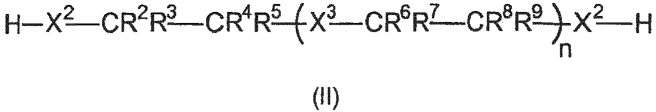 wherein
**X²** is independently for each occurrence selected from the group consisting of O and S;
**X³** is independently for each occurrence selected from the group consisting of O and S;
**R²**, **R³**, **R⁴**, **R⁵**, **R⁶**, **R⁷**, **R⁸**, and **R⁹** are independently for each occurrence selected from the group consisting of H, optionally substituted carbocyclyl and optionally substituted alkyl or any two of R², R³, R⁴, R⁵, R⁶, R⁷, R⁸, and R⁹ which are attached to the same carbon atom or two adjacent carbon atoms can be bonded to form a 3-to 7-membered carbocyclic ring;
**n** is independently for each occurrence selected from the group consisting of 0, 1, 2 and 3;
in the presence of at least one salt having the formula (IIIa)
M¹Y¹ (IIIa)
wherein
**M¹** is a cation; and
**Y¹** is a non-coordinating anion (NCA) selected from the group consisting of tetraarylborate anions B(Ar¹)₄, aluminate anions Al(OR¹⁰)₄ or (R¹⁰O)₃Al-F-Al(OR¹⁰)₃ and carborane anions R¹¹-CB₁₁R¹²₁₁;
wherein in the formula **B(Ar¹)₄**Ar¹ is independently for each occurrence selected from the group consisting of an optionally substituted aryl or optionally substituted heteroaryl group;
wherein in the formulae **Al(OR¹⁰)₄** or **(R¹⁰O)₃Al-F-Al(OR¹⁰)₃** R¹⁰ is independently for each occurrence selected from the group consisting of an optionally substituted alkyl group, wherein the optional substituent is selected from the group consisting of halogen, cyano, O-alkyl, and aryl which is optionally substituted by halogen, cyano, alkyl or CF₃;
wherein in the formula **R¹¹-[CB₁₁]R¹²₁₁** R¹¹ is selected from the group consisting of H, an aryl group and an alkyl group and R¹² is independently for each occurrence selected from the group consisting of H, halogen and an alkyl group, which is optionally substituted by halogen;
to form a NCA complex having the formula (V-i) and/or (V-ii)

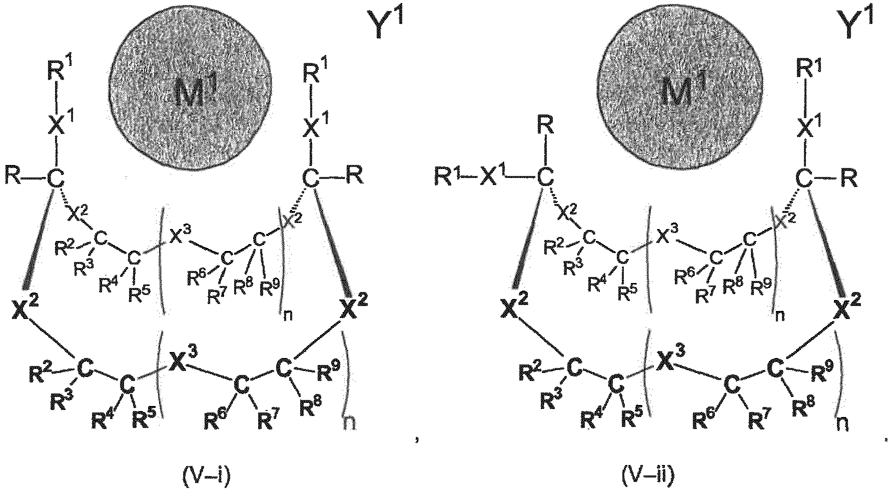

3. The method according to claim 1 or 2, further comprising the step of:
exchanging the anion Y¹ by the anion Y² in the NCA complex having the formula (IV), (V-i) and/or (V-ii), wherein **Y²** is an anion other than Y¹.

4. The method according to any one of claims 1 to 3, further comprising the step of:
exchanging the cation M¹ by the cation M² in the NCA complex having the formula (IV), (V-i) and/or (V-ii), wherein **M²** is a cation other than M¹.

5. The method according to claim 1 or 2, further comprising the step of removing the cation/anion pair M₁γ₁ from the NCA complex having the formula (IV), (V-i) and/or (V-ii).

6. The method according to any one of claims 1 to 5, wherein the step of reacting at least one compound having the formula (I) with at least one compound having the formula (II) is conducted in the presence of an acid, preferably trifluoroacetic acid.

7. The method according to claim 6, wherein the acid is added continuously or in incremental amounts, preferably in 1/n parts of the total amount, wherein n is from 2 to 100.

8. The method according to claim 6 or 7, wherein the concentration of the acid is in the range of 0.001 % to 1000 % by mole of the mole of the salt having the formula (IIIa).

9. The method according to any one of claims 1 to 8, wherein the step of reacting at least one compound having the formula (I) with at least one compound having the formula (II) is conducted under anhydrous conditions, preferably in the presence of a molecular sieve.

10. The method according to any one of claims 1 to 9, wherein the step of reacting at least one compound having the formula (I) with at least one compound having the formula (II) is conducted at a temperature in the range of -50 °C to 100 °C.

11. The method according to any one of claims 1 to 10, wherein the step of reacting at least one compound having the formula (I) with at least one compound having the formula (II) is conducted in an aprotic solvent, preferably a chlorohydrocarbon solvent.

12. The method according to any one of claims 1 to 10, wherein **Y¹** is a tetraarylborate anion B(Ar¹)₄.

13. A compound having the formula (IV): 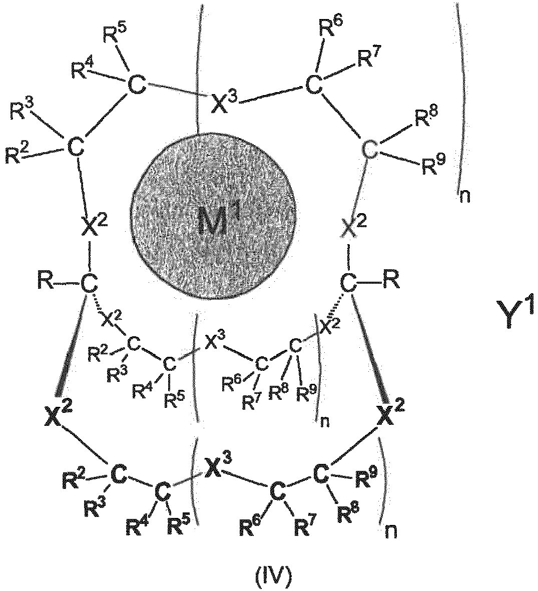 wherein X², X³, R, R², R³, R⁴, R⁵, R⁶, R⁷, R⁸, R⁹, n, M¹ and Y¹ are as defined in claim 1.

14. A compound having the formula (V-i) and/or (V-ii) 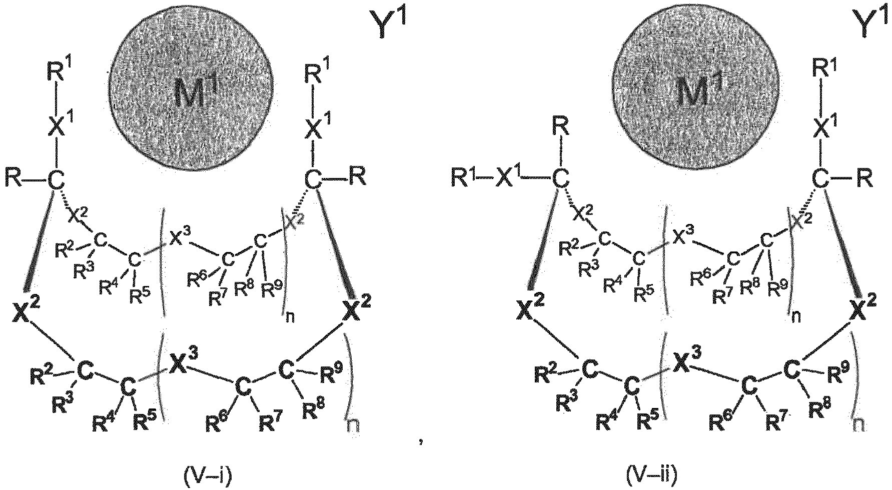 wherein X², X³, R, R², R³, R⁴, R⁵, R⁶, R⁷, R⁸, R⁹, n, M¹ and Y¹ are as defined in claim 1.

15. A pharmaceutical or diagnostic composition comprising a compound according to claim 13 or 14
wherein the pharmaceutical or diagnostic composition optionally further comprises a pharmaceutically or diagnostically acceptable excipient and/or carrier.

## Patentansprüche

1. Ein Verfahren, umfassend den Schritt des Umsetzens von
mindestens einer Verbindung mit der Formel (I) 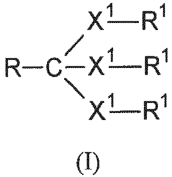 wobei
**X¹** unabhängig für jedes Vorkommen ausgewählt aus der Gruppe bestehend aus O und S steht;
**R¹** unabhängig für jedes Vorkommen ausgewählt aus der Gruppe bestehend aus Alkyl und Carbocyclyl steht; und
**R** für H oder einen organischen Rest steht;
mit mindestens einer Verbindung mit der Formel (II) 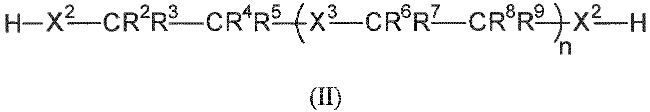 wobei
**X²** unabhängig für jedes Vorkommen ausgewählt aus der Gruppe bestehend aus O und S steht;
**X³** unabhängig für jedes Vorkommen ausgewählt aus der Gruppe bestehend aus O und S steht;
**R²**, **R³**, **R⁴**, **R⁵**, **R⁶**, **R⁷**, **R⁸** und **R⁹** unabhängig für jedes Vorkommen ausgewählt aus der Gruppe bestehend aus H, gegebenenfalls substituiertem Carbocyclyl und gegebenenfalls substituiertem Alkyl stehen oder beliebige
zwei Reste aus R², R³, R⁴, R⁵, R⁶, R⁷, R⁸ und R⁹, welche an das gleiche Kohlenstoffatom oder zwei benachbarte Kohlenstoffatome gebunden sind, verbunden sein können, um einen 3- bis 7-gliedrigen carbocyclischen Ring zu bilden;
**n** unabhängig für jedes Vorkommen ausgewählt aus der Gruppe bestehend aus 0, 1, 2 und 3 steht;
in Gegenwart von mindestens einem Salz mit der Formel (IIIa)
M¹Y¹ (IIIa)
wobei
**M¹** ein Kation ist; und
**Y¹** ein nicht-koordinierendes Anion (NCA) darstellt, ausgewählt aus der Gruppe bestehend aus Tetraarylboratanionen B(Ar¹)₄, Aluminatanionen Al(OR¹⁰)₄ oder (R¹⁰O)₃Al-F-Al(OR¹⁰)₃ und Carborananionen R¹¹-CB₁₁R¹²₁₁;
wobei in der Formel **B(Ar¹)₄** Ar¹ unabhängig für jedes Vorkommen ausgewählt aus der Gruppe bestehend aus einem gegebenenfalls substituierten Aryl- oder einer gegebenenfalls substituierten Heteroarylgruppe steht;
wobei in den Formeln **Al(OR¹⁰)₄** oder **(R¹⁰O)₃Al-F-Al(OR¹⁰)3** R¹⁰ unabhängig für jedes Vorkommen ausgewählt aus der Gruppe bestehend aus einer gegebenenfalls substituierten Alkylgruppe steht, wobei der optionale Substituent ausgewählt ist aus der Gruppe bestehend aus Halogen, Cyano, O-Alkyl und Aryl, welches gegebenenfalls mit Halogen, Cyano, Alkyl oder CF₃ substituiert steht;
wobei in der Formel **R¹¹-[CB₁₁]R¹²₁₁** R¹¹ ausgewählt ist aus der Gruppe bestehend aus H, einer Arylgruppe und einer Alkylgruppe und R¹² unabhängig für jedes Vorkommen ausgewählt aus der Gruppe bestehend aus H, Halogen und einer Alkylgruppe, welche gegebenenfalls mit Halogen substituiert ist, steht;
um einen NCA-Komplex mit der Formel (IV) zu bilden

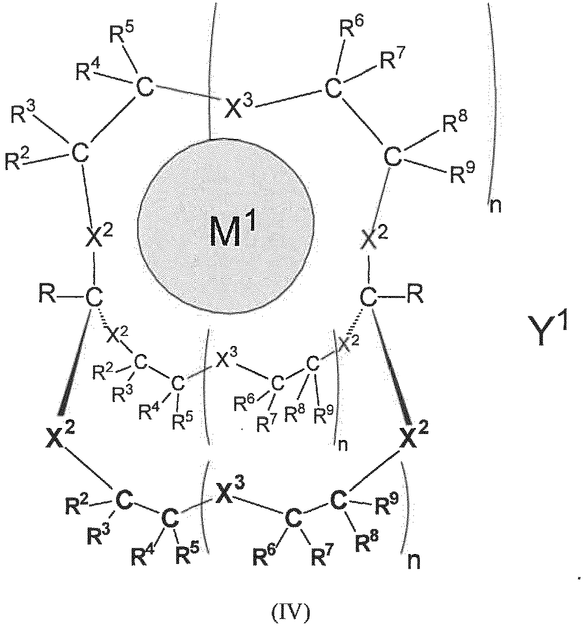

2. Ein Verfahren, umfassend den Schritt des Umsetzens von
mindestens einer Verbindung mit der Formel (I) 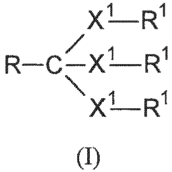 wobei
**X¹** unabhängig für jedes Vorkommen ausgewählt aus der Gruppe bestehend aus O und S steht;
**R¹** unabhängig für jedes Vorkommen ausgewählt aus der Gruppe bestehend aus Alkyl und Carbocyclyl steht; und
**R** für H oder einen organischen Rest steht;
mit mindestens einer Verbindung mit der Formel (II) 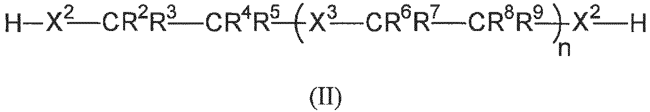 wobei
**X**² unabhängig für jedes Vorkommen ausgewählt aus der Gruppe bestehend aus O und S steht;
**X³** unabhängig für jedes Vorkommen ausgewählt aus der Gruppe bestehend aus O und S steht;
**R²**, **R³**, **R⁴**, **R⁵**, **R⁶**, **R⁷**, **R⁸** und **R⁹** unabhängig für jedes Vorkommen ausgewählt aus der Gruppe bestehend aus H, gegebenenfalls substituiertem Carbocyclyl und gegebenenfalls substituiertem Alkyl stehen oder beliebige zwei Reste R², R³, R⁴, R⁵, R⁶, R⁷, R⁸ und R⁹, welche an das gleiche Kohlenstoffatom oder zwei benachbarte Kohlenstoffatome gebunden sind, verbunden sein können, um einen 3- bis 7-gliedrigen carbocyclischen Ring zu bilden;
**n** unabhängig für jedes Vorkommen ausgewählt aus der Gruppe bestehend aus 0, 1, 2 und 3 steht;
in Gegenwart von mindestens einem Salz mit der Formel (IIIa)
M¹Y¹ (IIIa)
wobei
**M¹** ein Kation. ist; und
**Y¹** ein nicht-koordinierendes Anion (NCA) darstellt, ausgewählt aus der Gruppe bestehend aus Tetraarylboratanionen B(Ar¹)₄, Aluminatanionen Al(OR¹⁰)₄ oder (R¹⁰O)₃Al-F-Al(OR¹⁰)₃ und Carborananionen R¹¹-CB₁₁R¹²₁₁; wobei in der Formel **B(Ar¹)₄** Ar¹ unabhängig für jedes Vorkommen ausgewählt aus der Gruppe bestehend aus einer gegebenenfalls substituierten Aryl- oder gegebenenfalls substituierten Heteroarylgruppe steht;
wobei in den Formeln **Al(OR¹⁰)₄** oder **(R¹⁰O)₃Al-F-Al(OR¹⁰)₃** R¹⁰ unabhängig für jedes Vorkommen ausgewählt aus der Gruppe bestehend aus einer gegebenenfalls substituierten Alkylgruppe steht, wobei der optionale Substituent ausgewählt ist aus der Gruppe bestehend aus Halogen, Cyano, O-Alkyl und Aryl, welches gegebenenfalls mit Halogen, Cyano, Alkyl oder CF₃ substituiert steht;
wobei in der Formel **R¹¹-[CB_{11]}R¹²₁₁** R¹¹ ausgewählt ist aus der Gruppe bestehend aus H, einer Arylgruppe und einer Alkylgruppe, und R¹² unabhängig für jedes Vorkonunen ausgewählt aus der Gruppe bestehend aus H, Halogen und einer Alkylgruppe, welche gegebenenfalls mit Halogen substituiert ist, steht;
um einen NCA-Komplex mit der Formel (V-i) und/oder (V-ii) zu bilden

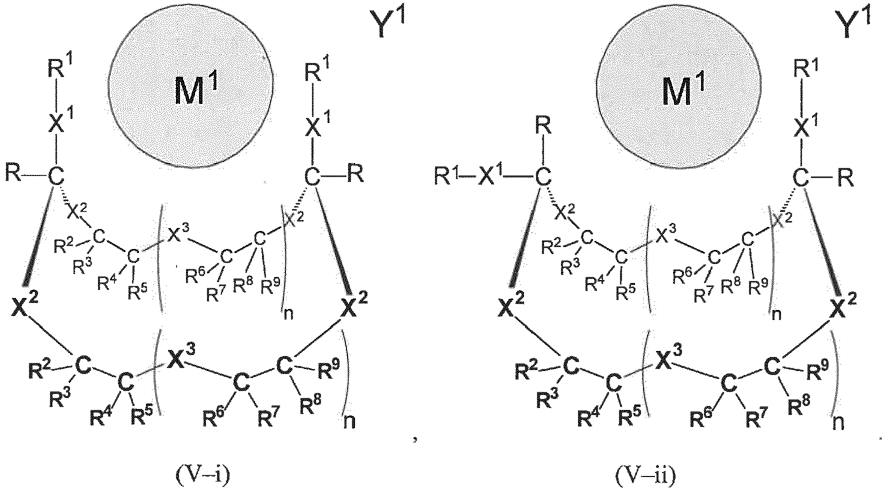

3. Das Verfahren gemäß Anspruch 1 oder 2, ferner umfassend den Schritt:
Austauschen des Anions Y¹ durch das Anion Y² in dem NCA-Komplex mit der Formel (IV), (V-i) und/oder (V-ii), wobei **Y²** ein von Y¹ verschiedenes Anion mit Ausnahme von Y¹ ist.

4. Das Verfahren gemäß einem der Ansprüche 1 bis 3, ferner umfassend den Schritt:
Austauschen des Kations M¹ durch das Kation M² in dem NCA-Komplex mit der Formel (IV), (V-i) und/oder (V-ii), wobei **M²** ein von M¹ verschiedenes Kation mit Ausnahme von M¹ ist.

5. Das Verfahren gemäß Anspruch 1 oder 2, ferner umfassend den Schritt des Entfernens des Kationen-/Anionenpaars M¹Y¹ aus dem NCA-Komplex mit der Formel (IV), (V-i) und/oder (V-ii).

6. Das Verfahren gemäß einem der Ansprüche 1 bis 5, wobei der Schritt des Umsetzens mindestens einer Verbindung mit der Formel (I) mit mindestens einer Verbindung mit der Formel (II) in Gegenwart einer Säure, vorzugsweise Trifluoressigsäure, durchgeführt wird.

7. Das Verfahren gemäß Anspruch 6, wobei die Säure fortlaufend oder in fortlaufend steigender Menge, vorzugsweise in 1/n Teilen der Gesamtmenge, wobei n gleich 2 bis 100 ist, zugeführt wird.

8. Das Verfahren gemäß Anspruch 6 oder 7, wobei die Konzentration der Säure im Bereich von 0,001 Mol-% bis 1000 Mol% des Mols des Salzes mit der Formel (IIIa) liegt.

9. Das Verfahren gemäß einem der Ansprüche 1 bis 8, wobei der Schritt des Umsetzens mindestens einer Verbindung mit der Formel (I) mit mindestens einer Verbindung mit der Formel (II) unter wasserfreien Bedingungen, vorzugsweise in Gegenwart eines Molekularsiebs, durchgeführt wird.

10. Das Verfahren gemäß einem der Ansprüche 1 bis 9, wobei der Schritt des Umsetzens mindestens einer Verbindung mit der Formel (I) mit mindestens einer Verbindung mit der Formel (II) bei einer Temperatur im Bereich von -50 °C bis 100 °C durchgeführt wird.

11. Das Verfahren gemäß einem der Ansprüche 1 bis 10, wobei der Schritt des Umsetzens mindestens einer Verbindung mit der Formel (I) mit mindestens einer Verbindung mit der Formel (II) in einem aprotischen Lösungsmittel, vorzugsweise einem Chlorkohlenwasserstoff Lösungsmittel, durchgeführt wird.

12. Das Verfahren gemäß einem der Ansprüche 1 bis 10, wobei Y¹ ein Tetraarylboratanion B(Ar¹)₄ ist.

13. Eine Verbindung mit der Formel (IV): 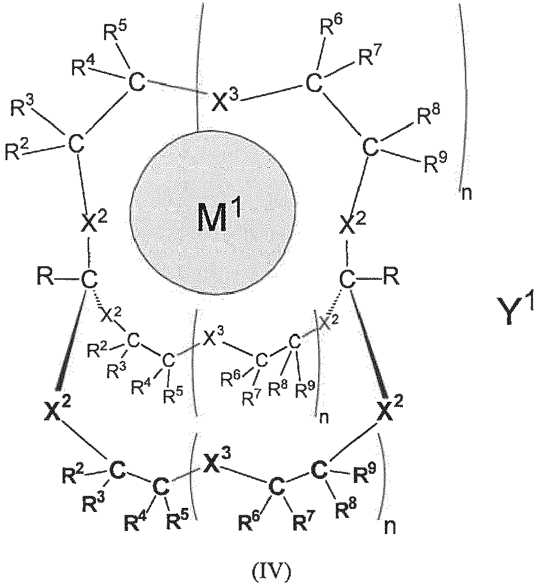 wobei X², X³, R, R², R³, R⁴, R⁵, R⁶, R⁷, R⁸, R⁹, n, M¹ und Y¹ wie in Anspruch 1 definiert sind.

14. Eine Verbindung mit der Formel (V-i) und/oder (V-ii) 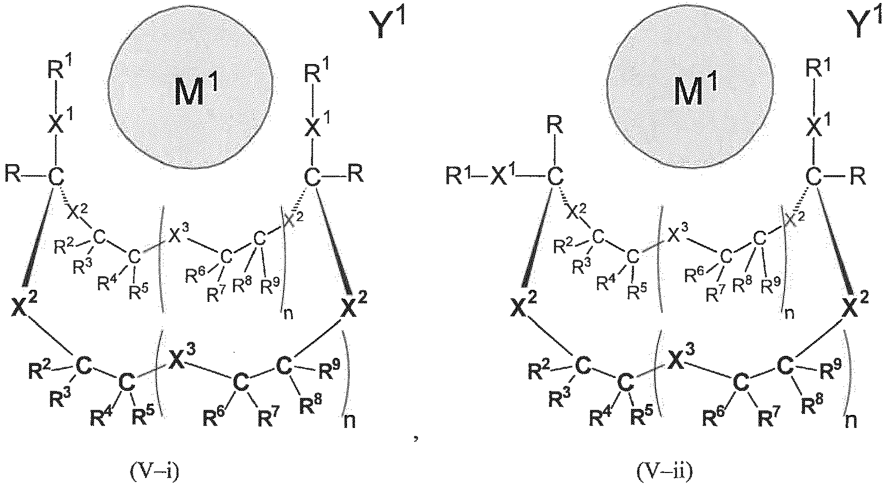 wobei X² X³, R, R² R³ R⁴ R⁷, R⁶ R⁷ R⁸ R⁹ n, M¹ und Y¹ wie in Anspruch 1 definiert sind.

15. Ein Arzneimittel oder eine diagnostische Zusammensetzung, umfassend eine Verbindung gemäß Anspruch 13 oder 14, wobei das Arzneimittel oder die diagnostische Zusammensetzung gegebenenfalls ferner einen pharmazeutisch oder diagnostisch verträglichen Exzipienten und/oder Träger umfasst.

## Revendications

1. Méthode comprenant l'étape de réaction
d'au moins un composé ayant la formule (I) 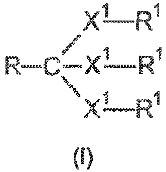 dans laquelle
X¹ est choisi indépendamment pour chaque occurrence dans le groupe constitué de O et S;
R¹ est choisi indépendamment pour chaque occurrence dans le groupe constitué des groupes alkyle et carbocyclyle ; et
R représente H ou une fraction organique ;
avec au moins un composé ayant la formule (II) 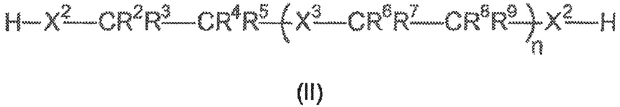 dans laquelle
X² est choisi indépendamment pour chaque occurrence dans le groupe constitué de O et S;
X³ est choisi indépendamment pour chaque occurrence dans le groupe constitué de O et S;
R², R³, R⁴, R⁵, R⁶, R⁷, R⁸, et R⁹ sont choisis indépendamment pour chaque occurrence dans le groupe constitué de H, de groupes carbocyclyle éventuellement substitué et alkyle éventuellement substitué ou deux quelconques de R², R³, R⁴, R⁵, R⁶, R⁷, R⁸, et R⁹ qui sont fixés au même atome de carbone ou à deux atomes de carbone adjacents peuvent être liés pour former un cycle carbocyclique de 3 à 7 chaînons ;
n est choisi indépendamment pour chaque occurrence dans le groupe constitué de 0, 1, 2 eut 3 ;
en présence d'au moins un sel ayant la formule (IIIa)
M¹Y¹ (IIIa)
dans laquelle
M¹ représente un cation ; et
Y¹ représente un anion non coordonnant (NCA) choisi dans le groupe constitué d'anions de tétraborate d'aryle B(Ar¹)₄, d'anions d'aluminate (Al(OR¹⁰)₄ ou (R¹⁰O)₃Al-F-Al(OR¹⁰)₃ et d'anions de carborane R¹¹-CB₁₁R¹²₁₁ ;
dans laquelle dans la formule B(Ar¹)₄ Ar¹ est choisi indépendamment pour chaque occurrence dans le groupe constitué d'un groupe aryle éventuellement substitué ou hétéroaryle éventuellement substitué ;
dans laquelle dans les formules Al(OR¹⁰)₄ ou (R¹⁰O)₃Al-F-Al(OR¹⁰)₃ R¹⁰ est choisi indépendamment pour chaque occurrence dans le groupe constitué d'un groupe alkyle éventuellement substitué, dans lequel le substituant éventuel est choisi dans le groupe constitué d'un atome d'halogène, des groupes cyano, O-alkyle et aryle qui est éventuellement substitué par un atome d'halogène, un groupe cyano, alkyle ou CF₃ ;
dans laquelle dans la formule R¹¹-[CB₁₁]R¹²₁₁ R¹¹ est choisi dans le groupe constitué de H, un groupe aryle et un groupe alkyle et R¹² est choisi indépendamment pour chaque occurrence dans le groupe constitué de H, un atome d'halogène et un groupe alkyle, qui est éventuellement substitué par un atome d'halogène ;
pour former un complexe du NCA ayant la formule (IV)

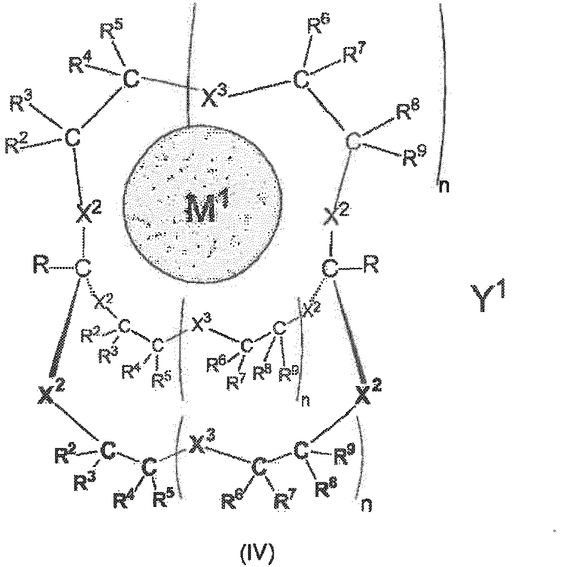

2. Méthode comprenant l'étape de réaction
d'au moins un composé ayant la formule (I) 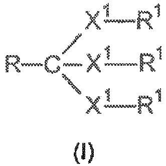 dans laquelle
X¹ est choisi indépendamment pour chaque occurrence dans le groupe constitué deOetS;
R¹ est choisi indépendamment pour chaque occurrence dans le groupe constitué de groupes alkyle et carbocyclyle ; et
R représente H ou une fraction organique ;
avec au moins un composé ayant la formule (II) 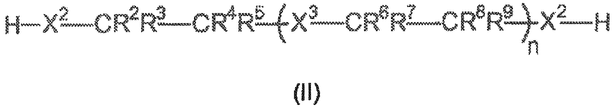 dans laquelle
X² est choisi indépendamment pour chaque occurrence dans le groupe constitué de O et S;
X³ est choisi indépendamment pour chaque occurrence dans le groupe constitué de O et S;
R², R³, R⁴, R⁵, R⁶, R⁷, R⁸, et R⁹ sont choisis indépendamment pour chaque occurrence dans le groupe constitué de H, de groupes carbocyclyle éventuellement substitué et alkyle éventuellement substitué ou deux quelconques de R², R³, R⁴, R⁵, R⁶, R⁷, R⁸, et R⁹ qui sont fixés au même atome de carbone ou à deux atomes de carbone adjacents peuvent être liés pour former un cycle carbocyclique de 3 à 7 chaînons ;
n est choisi indépendamment pour chaque occurrence dans le groupe constitué de 0, 1, 2 et 3 ;
en présence d'au moins un sel ayant la formule (IIIa)
M¹Y¹ (IIIa)
dans laquelle
M¹ représente un cation ; et
Y¹ représente un anion non coordonnant (NCA) choisi dans le groupe constitué d'anions de tétraborate d'aryle B(Ar¹)₄, d'anions d'aluminate (Al(OR¹⁰)₄ ou (R¹⁰O)₃Al-F-Al(OR¹⁰)₃ et d'anions de carborane R¹¹-CB₁₁R¹²₁₁ ;
dans laquelle dans la formule B(Ar¹)₄ Ar¹ est choisi indépendamment pour chaque occurrence dans le groupe constitué d'un groupe aryle éventuellement substitué ou hétéroaryle éventuellement substitué ;
dans laquelle dans les formules Al(OR¹⁰)₄ ou (R¹⁰O)₃Al-F-Al(OR¹⁰)₃ R¹⁰ est choisi indépendamment pour chaque occurrence dans le groupe constitué d'un groupe alkyle éventuellement substitué, dans lequel le substituant éventuel est choisi dans le groupe constitué d'un atome d'halogène, des groupes cyano, O-alkyle et aryle qui est éventuellement substitué par un atome d'halogène, un groupe cyano, alkyle ou CF₃ ;
dans laquelle dans la formule R¹¹-[CB_{n]}R¹²₁₁ R¹¹ est choisi dans le groupe constitué de H, un groupe aryle et un groupe alkyle et R¹² est choisi indépendamment pour chaque occurrence dans le groupe constitué de H, un atome d'halogène et un groupe alkyle, qui est éventuellement substitué par un atome d'halogène ;
pour former un complexe du NCA ayant la formule (V-i) et/ou (V-ii)

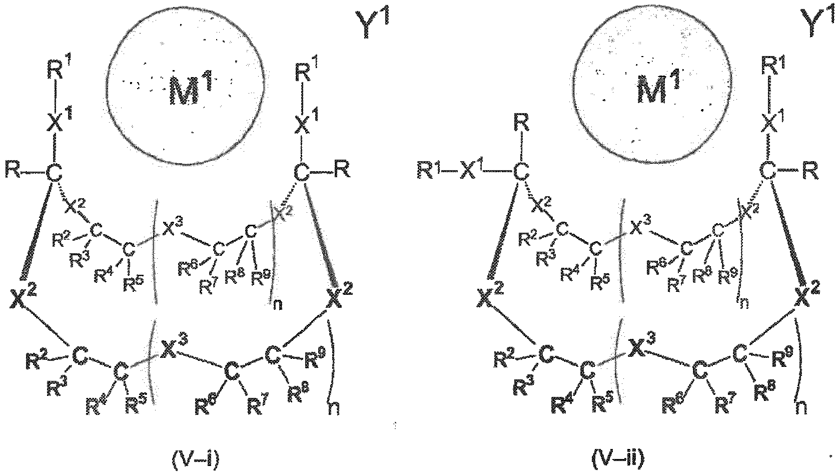

3. Méthode selon la revendication 1 ou 2, comprenant en outre l'étape suivante :
l'échange de l'anion Y¹ par l'anion Y² dans le complexe du NCA ayant la formule (IV), (V-i) et/ou (V-ii), dans lequel Y² est un anion différent de Y¹.

4. Méthode selon l'une quelconque des revendications 1 à 3, comprenant en outre l'étape suivante :
l'échange du cation M¹ par le cation M² dans le complexe du NCA ayant la formule (IV), (V-i) et/ou (V-ii), dans lequel M² est un cation différent de M¹.

5. Méthode selon la revendication 1 ou 2, comprenant en outre l'étape d'élimination de la paire cation/anion M¹Y¹ à partir du complexe du NCA ayant la formule (IV), (V-i) et/ou (V-ii).

6. Méthode selon l'une quelconque des revendications 1 à 5, dans laquelle l'étape de réaction d'au moins un composé ayant la formule (I) avec au moins un composé ayant la formule (II) est conduite en présence d'un acide, de préférence l'acide trifluoroacétique.

7. Méthode selon la revendication 6, dans laquelle l'acide est ajouté en continu ou en quantités incrémentielles, de préférence en 1/n parties de la quantité totale, dans laquelle n vaut de 2 à 100.

8. Méthode selon la revendication 6 ou 7, dans laquelle la concentration de l'acide se situe dans la plage de 0,001 % à 1000 % par mole de la mole du sel ayant la formule (IIIa).

9. Méthode selon l'une quelconque des revendications 1 à 8, dans laquelle l'étape de réaction d'au moins un composé ayant la formule (I) avec au moins un composé ayant la formule (II) est conduite dans des conditions anhydres, de préférence en présence d'un tamis moléculaire.

10. Méthode selon l'une quelconque des revendications 1 à 9, dans laquelle l'étape de réaction d'au moins un composé ayant la formule (I) avec au moins un composé ayant la formule (II) est conduite à une température située dans la plage de -50 °C à 100 °C.

11. Méthode selon l'une quelconque des revendications 1 à 10, dans laquelle l'étape de réaction d'au moins un composé ayant la formule (I) avec au moins un composé ayant la formule (II) est conduite dans un solvant aprotique, de préférence un solvant d'hydrocarbure chloré.

12. Méthode selon l'une quelconque des revendications 1 à 10, dans laquelle Y¹ représente un anion de tétraborate d'aryle B(Ar¹)₄.

13. Composé ayant la formule (IV) : 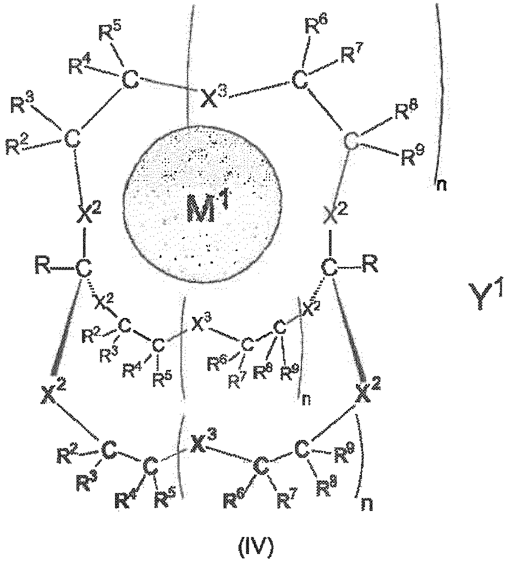 dans laquelle X² X³, R, R², R³, R⁴, R⁵, R⁶, R⁷, R⁸, R⁹, n, M¹ et Y¹ sont tels que définis dans la revendication 1.

14. Composé ayant la formule (V-i) et/ou (V-ii) 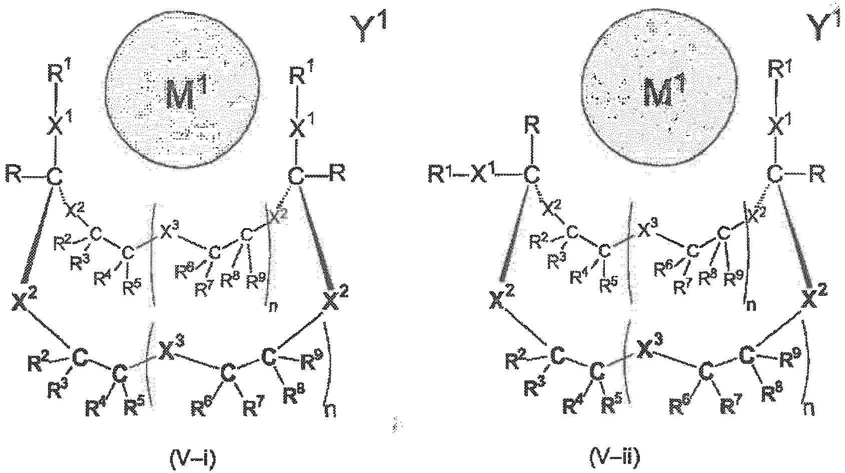 dans lesquelles X², X³, R, R⁷, R³, R⁴, R⁷, R⁶, R⁷, R⁸, R⁹, n, M¹ et Y¹ sont tels que définis dans la revendication 1.

15. Composition pharmaceutique ou diagnostique comprenant un composé selon la revendication 13 ou 14, dans laquelle la composition pharmaceutique ou diagnostique comprend éventuellement en outre un excipient et/ou un support acceptable d'un point de vue pharmaceutique ou diagnostique.
